# EUROPEAN PATENT APPLICATION

(11) **EP 4 729 541 A1**
(43) Date of publication of application: **22.04.2026**
(21) Application number: 24206957.3
(22) Date of filing: 16.10.2024
(51) Int. Cl.: C07K 16/28, A61P 5/00, A61P 17/06, A61P 29/00

(54) **ANTI- IGFBP7 ANTIBODIES AND USES THEREOF**

(71) Applicant: Fiorina, Paolo, 20054 Milano Due, Segrate (MI) (IT)
(72) Inventor: Fiorina, Paolo, 20054 Milano Due, Segrate (MI) (IT)
(74) Representative: Pace Napoleone, Maria

(57) **Abstract**

The present invention relates to an antibody or antigen binding fragment thereof that binds specifically to IGFBP7 and has pro-survival activity, decreases stress-mediated senescence/inflammation and pro-fibrotic tissue injury. In particular, the invention relates to antibodies or antigen binding fragments thereof that bind with high affinity and specificity to human IGFBP7 and/or to at least one of its receptors.

## Description

### TECHNICAL FIELD

The present invention relates to an antibody or antigen binding fragment thereof that binds specifically to IGFBP7 and has pro-survival activity, decreases stress-mediated senescence/inflammation and pro-fibrotic tissue injury. In particular, the invention relates to antibodies or antigen binding fragments thereof that bind with high affinity and specificity to human IGFBP7 and/or to at least one of its receptors.

### BACKGROUND OF THE INVENTION

### Insulin-like growth factor binding protein 7

Insulin-like growth factor binding protein 7 (IGFBP7) is the first member identified among the Insulin-like growth factor binding proteins (IGFBPs), a family of low-affinity IGFBPs termed IGFBP-rp1-10 capable to bind, carry and modulate Insulin-like growth factors (IGFs) availability in the circulation (Shibata et al., 2004). It was initially named IGFBP7 because of its capability to attach IGFs through the N-terminal domain (Yamanaka et al., 1997), which contains an IGFBP motif (GCGCCXXC (SEQ ID NO. 127); wherein the "X" can be any amino acid) in a domain including 12 conserved amino acids (cysteines). However, the C-terminus of IGFBP7 differs substantially from the other IGFBPs because it lacks conserved cysteines (Oh et al., 1996) and IGFBP7 has a 100-fold lower affinity for IGF-1 than the other IGFBPs. Besides IGF-specific mechanisms, IGFBP7 has roles also in other activities, which are IGFs independent. It can bind to activin A, and thus it may act to modulate the effects related to the TGF-β superfamily, including suppressing proliferation and growth (van Eikema Hommes et al., 2000). Expression of IGFBP7 has been shown to be upregulated in cells treated with TGF-β1 and retinoic acid (RA) (Swisshelm et al., 1995). Furthermore, IGFBP7 was also discovered to bind to type IV collagen (Akaogi et al., 1996; St. Croix et al., 2000). IGFBP7 has been demonstrated to bind also to CD93, which is mainly expressed on endothelial cells, and the signaling of IGFBP7 through CD93 is involved in modulating neoangiogenesis in tumor growth (Li Y et al., 2023). Interestingly, in breast cancer cells, IGFBP7 may bind also to IGF-1 receptor when it is not occupied by IGF-1 or IGF-2 to block and modulate the IGF-1/2 activity. The resulting prolonged exposure to IGFBP7 renders the IGF-1 receptor positive cells more sensible to apoptosis and cell death (Edvokimova et al., 2012). IGFBP7 has a molecular weight of 29 kDa and it is primarily expressed in liver, bone, muscle, heart, and kidney, particularly in the tubular compartment (https://www.proteinatlas.org/ENSG00000163453-IGFBP7/tissue). It also circulates in the bloodstream as a carrier for IGF proteins and its levels have been recently linked to the risk of developing acute kidney injury (AKI), thereby establishing a role for IGFBP7 as a peripheral prognostic biomarker for kidney damage (Bai et al., 2018). The use of IGFBP7 levels as a diagnostic tool has been reported in major papers describing the results of the Sapphire and of the Topaz studies (Kashani et al., 2013, Koyner et al. 2015), both supporting the idea that elevated levels of IGFBP7 are associated with high risk for developing moderate to severe AKI (Bihorac et al., 2014) and opening the path for US Food and Drug Administration (FDA) to approve IGFBP7 and its binding partner TIMP2 as biomarker for risk assessment of AKI in intensive care unit (ICU) patients in 2014 (US Food and Drug Administration, 2014). Despite this, the role of IGFBP7 in directly mediating kidney damage has been only partially explored. It has been demonstrated that IGFBP7 promotes kidney tubular cell damage at the early stage in an autocrine fashion, by blocking the cell cycle and promoting cell apoptosis (Kashani et al., 2013; Wang et al., 2018). However, the mechanisms behind this effect on tubular cells are still under investigation and the protective effect that IGFBP7 inhibition may have in preventing cell damage and death in health/disease conditions is still unexplored.

### IGFBP7 signaling in disease conditions

### IGFBP7 in Acute Kidney Injury (AKI)

Over the years there have been numerous studies with the aim to identify novel biomarkers for early detection of AKI in order to avoid poor prognosis.

Among all markers studied, tissue inhibitor of metalloproteinases-2 (TIMP-2) and IGFBP7 have been found as the most accurate indicators for risk of developing AKI. For this reason, in 2014, the Food and Drug Administration (FDA) approved the analysis of TIMP-2 - IGFBP7 blood level to be used in ICU patients to predict the risk of developing moderate to severe AKI within 24 hours (Xie et al., 2019).

Recently, it has been shown that IGFBP7 overexpression in the tubular cell line HK2 promotes cisplatin-induced inflammation cytokines, possibly via induction of programmed cell death and p65 NF-kB phosphorylation. In addition, IGFBP7 reduces Poly(ADP)-ribose polymerase (PARP1) protein degradation by binding to its BRCT domain and antagonizing ubiquitination from E3 ligase RNF4. Consistently, disruption of PARP1 attenuates cisplatin-induced inflammation, by possibly linking inflammation promotion with excessive activity of PARP1 due to its reduced turn-over. Thus, IGFBP7 in circulation acts as a biomarker and key mediator of AKI by inhibiting RNF4/PARP1-mediated tubular injury and inflammation (Yu et al., 2022). Interestingly, genetic inhibition of IGFBP7 offered a significant protection from AKI both *in vitro* and *in vivo* (Yu et al., 2022). The detrimental effect observed *in vitro* in tubular cells, including cell damage and/or death and tissue fibrosis, induced by inflammatory stimuli (lipopolysaccharide, LPS) with IGFBP7, reinforced the negative role for IGFBP7 during stress and inflammation. The fact that the neutralization of IGFBP7 with siRNA was able to counteract the cell damage further confirmed the protective effect that IGFBP7 blockade may exert also as a therapeutic approach.

### IGFBP7 in Heart Failure (HF)

A proteomic analysis conducted in a murine model of chronic heart failure (HF) identified IGFBP7 as a candidate biomarker for this disease condition. Based on this observation, elevated levels of IGFBP7 were measured in patients with chronic HF and were associated with the development of major adverse cardiovascular events (Chugh et al., 2013; Motiwala et al., 2014).

In a recent study, it has been shown that IGFBP7 promoted cardiac senescence by stimulating IGF-1R/IRS/AKT-dependent suppression of FOXO3a, preventing DNA repair and reactive oxygen species (ROS) detoxification, thereby accelerating the progression of HF (Zhang et al., 2022).

Furthermore, levels of IGFBP7 were found to correlate with the risk of developing HF in a cohort of patients followed up for prevention studies (Bracun et al., 2022). Further proteomic analysis demonstrated that among different proteins correlating with the risk of developing HF, IGFBP7 was evident in serum of patients at higher risk and further supported the hypothesis of a senescence-mediated proapoptotic mechanism acting through IGFBP7 in heart disease. Despite these interesting observations, no further explorations have been conducted in addressing whether IGFBP7 has a causative role in the development of HF in humans and whether blockade of IGFBP7 was able to rescue the negative effect observed. Some *in vivo* data recently demonstrated that IGFBP7 inhibition with a rabbit monoclonal antibody protected vascular damage and prevented heart injury, including HF, in a murine model in which HF was induced by pressure overload (Zhang et al., Nat Cardiovascular Res 2022). It also suggested that IGFBP7 inhibition may have some effect in protecting cardiac tissue from stress and hypertrophy. This reinforced the relevance of IGFBP7 and its blockade in cardiovascular remodeling, stress and vascular fibrosis.

### IGFBP7 in inflammation and vascular damage

Psoriasis is a common T cell-mediated inflammatory skin disease characterized by tortuous and dilated skin vessels. The main cause of this disease is loosening of glycocalyx integrity, which is a structure formed by glycoproteins covering the inner wall of blood vessels, and together with cellular junctions of endothelial cells (ECs), it forms the endothelial barrier (Jourde-Chiche et al., 2019). Loss of this structure integrity promotes T cell extravasation and cutaneous inflammation. IGFBP7 is one of the main upregulated transcripts and proteins in psoriatic ECs and it determines glycocalyx shedding by binding to one of its main components, the heparan sulfate (HS), thus exposing the adhesion molecules underneath and driving T cell extravasation into skin lesions (Q. Li et al., 2023). Elevated IGFBP7 levels have also been documented in patients with psoriasis as compared to healthy subjects, thus supporting the role of IGFBP7 in this disease condition (Jourde-Chiche et al., 2019).

Importantly, pharmacological blockade of IGFBP7 *in vivo* with a rabbit monoclonal antibody commercially available (A4615, ABclonal) in a psoriasis-like mouse model was associated with an improvement of skin inflammation and a recovery of ECs structure and function (Q. Li et al., 2023). The data further suggested that upregulated IGFBP7 expression in ECs is linked to the development of inflammation and vascular abnormalities and that targeting IGFBP7 may represent a therapeutic strategy for psoriasis and its complications.

There is still the need in the art for anti-IGFBP7 antibodies that bind with high affinity and specificity to human IGFBP7 and/or to at least one of its receptors, and ensure cellular survival, reduce stress-mediated senescence/inflammation and pro-fibrotic tissue injury.

### SUMMARY OF THE INVENTION

The present invention discloses inhibitors of IGFBP7 and/or of at least one of its receptors. Said inhibitors have pro-survival activity and decrease stress-mediated senescence/inflammation and pro-fibrotic tissue injury.

In particular, said inhibitors disclosed herein are antibody molecules (preferably human or humanized antibody molecules) or antigen binding fragments that bind to human and/or mouse IGFBP7 or to at least one of its receptors with high specificity.

Advantageously said antibodies or antigen binding fragments of the present invention bind to human IGFBP7 and/or to at least one of its receptors with high affinity and specificity compared to monoclonal and polyclonal antibodies in the prior art.

Said antibody molecules inhibit stress-mediated cell senescence, damage, inflammation and tissue fibrosis.

Preferably the antibody of the present invention is for use in the treatment and/or prevention of vascular stress-mediated senescence, inflammation and fibrosis (psoriasis, heart failure, vasculopathy).

Preferably the antibody of the present invention is for use in the treatment and/or prevention also of acute stress cellular damage, preferably including acute kidney injury.

Immunoconjugates, multi- or bispecific antibody molecules and pharmaceutical compositions comprising the antibody molecules are also subject matter of the present invention.

Therefore, in a first aspect, it is an object of the invention isolated antibody or antigen binding fragment or variant thereof that binds to IGFBP7 and/or receptor thereof. Preferably, isolated antibody or antigen binding fragment thereof comprises:
a. a heavy chain variable domain (VH) comprising:
   i. a CDR1 sequence of the amino acid sequence selected from the group consisting of: SEQ ID NO: 1, 14, 29, 32, 9, 20, 24 and 26;
   ii. a CDR2 sequence of the amino acid sequence selected from the group consisting of: SEQ ID NO: 2, 15, 30, 33, 13, 10, 17, 21 and 27; and
   iii. a CDR3 sequence of the amino acid sequence selected from the group consisting of: SEQ ID NO: 8, 4, 3, 16, 31, 34, 128, 5, 6, 7, 11, 12, 18, 19, 22, 23, 25 and 28; and/or
b. a light chain variable domain (VL) comprising:
   i. a CDR1 sequence of the amino acid sequence selected from the group consisting of: SEQ ID NO: 35, 38, 41, 70, 53, 47, 50, 57, 60 and 66;
   ii. a CDR2 sequence of the amino acid sequence selected from the group consisting of: SEQ ID NO: 36, 39, 42, 71, 54, 48, 51, 58, 61 and 67; and
   iii. a CDR3 sequence of the amino acid sequence selected from the group consisting of: SEQ ID NO: 46, 40, 37, 56, 69, 72, 55, 43, 44, 45, 49, 52, 59, 62, 63, 64, 65 and 68; and
wherein CDRs are according to Kabat.

Still preferably, the isolated antibody or antigen binding fragment thereof comprises:
a. a heavy chain variable domain (VH) comprising:
   i. a CDR1 sequence of the amino acid sequence selected from the group consisting of: SEQ ID NO: 1, 14, 29, and 32;
   ii. a CDR2 sequence of the amino acid sequence selected from the group consisting of: SEQ ID NO: 2, 15, 30 and 33; and
   iii. a CDR3 sequence of the amino acid sequence selected from the group consisting of: SEQ ID NO: 8, 4, 3, 16, 31 and 34; and/or
b. a light chain variable domain (VL) comprising:
   i. a CDR1 sequence of the amino acid sequence selected from the group consisting of: SEQ ID NO: 35, 38, 41 and 70;
   ii. a CDR2 sequence of the amino acid sequence selected from the group consisting of: SEQ ID NO: 36, 39, 42 and 71; and
   iii. a CDR3 sequence of the amino acid sequence selected from the group consisting of: SEQ ID NO: 46, 40, 37, 56, 69 and 72; and
wherein CDRs are according to Kabat.

Preferably, the isolated antibody or antigen binding fragment thereof comprises as CDRs:
SEQ ID NO: 1 and SEQ ID NO: 2 and SEQ ID NO: 8 and SEQ ID NO: 35 and SEQ ID NO: 36 and SEQ ID NO: 46 of YU1425-A10; or
SEQ ID NO: 1 and SEQ ID NO: 2 and SEQ ID NO: 4 and SEQ ID NO: 38 and SEQ ID NO: 39 and SEQ ID NO: 40 of YU1425-A09; or
SEQ ID NO: 1 and SEQ ID NO: 2 and SEQ ID NO: 3 and SEQ ID NO: 35 and SEQ ID NO: 36 and SEQ ID NO: 37 of YU1425-E10; or
SEQ ID NO: 14 and SEQ ID NO: 15 and SEQ ID NO: 16 and SEQ ID NO: 38 and SEQ ID NO: 39 and SEQ ID NO: 56 of YU1425-C09; or
SEQ ID NO: 29 and SEQ ID NO: 30 and SEQ ID NO: 31 and SEQ ID NO: 41 and SEQ ID NO: 42 and SEQ ID NO: 69 of YU1425-C01; or
SEQ ID NO: 32 and SEQ ID NO: 33 and SEQ ID NO: 34 and SEQ ID NO: 70 and SEQ ID NO: 71 and SEQ ID NO: 72 of YU1425-E01.

Preferably, the isolated antibody or antigen binding fragment thereof comprises:
a. a heavy chain variable domain sequence having at least 80 %, or 85 %, or 90%, or 95%, or 100% identity to the amino acid sequence selected from the group consisting of: SEQ ID NO: 78, 74, 73, 82, 89 and 90;
b. a light chain variable domain sequence having at least 80 %, or 85 %, or 90%, or 95%, or 100% identity to the amino acid sequence selected from the group consisting of: SEQ ID NO: 96, 92, 91, 100, 107 and 108; or
c. the heavy chain variable domain of (a) and the light chain variable domain of (b).

Still preferably, the isolated antibody or antigen binding fragment thereof comprises:
a heavy chain variable domain comprising or consisting of a sequence having at least 80 %, or 85 %, or 90%, or 95%, or 100% identity to SEQ ID NO: 78 and a light chain variable domain comprising or consisting of a sequence having at least 80 %, or 85 %, or 90%, or 95%, or 100% identity to SEQ ID NO: 96;
a heavy chain variable domain comprising or consisting of a sequence having at least 80 %, or 85 %, or 90%, or 95%, or 100% identity to SEQ ID NO: 74 and a light chain variable domain comprising or consisting of a sequence having at least 80%, or 85 %, or 90%, or 95%, or 100% identity to SEQ ID NO: 92;
a heavy chain variable domain comprising or consisting of a sequence having at least 80%, or 85 %, or 90%, or 95%, or 100% identity to SEQ ID NO: 73 and a light chain variable domain comprising or consisting of a sequence having at least 80%, or 85 %, or 90%, or 95%, or 100% identity to SEQ ID NO: 91;
a heavy chain variable domain comprising or consisting of a sequence having at least 80%, or 85 %, or 90%, or 95%, or 100% identity to SEQ ID NO: 82 and a light chain variable domain comprising or consisting of a sequence having at least 80%, or 85 %, or 90%, or 95%, or 100% identity to SEQ ID NO: 100;
a heavy chain variable domain comprising or consisting of a sequence having at least 80%, or 85 %, or 90%, or 95%, or 100% identity to SEQ ID NO: 89 and a light chain variable domain comprising or consisting of a sequence having at least 80%, or 85 %, or 90%, or 95%, or 100% identity to SEQ ID NO: 107; or
a heavy chain variable domain comprising or consisting of a sequence having at least 80%, or 85 %, or 90%, or 95%, or 100% identity to SEQ ID NO: 90 and a light chain variable domain comprising or consisting of a sequence having at least 80%, or 85 %, or 90%, or 95%, or 100% identity to SEQ ID NO: 108.

Still preferably, the isolated antibody or antigen binding fragment thereof comprises:
a. a heavy chain variable domain (VH) comprising:
   - a CDR1 comprising or consisting of the amino acid sequence of SEQ ID NO: 1;
   - a CDR2 comprising or consisting of the amino acid sequence SEQ ID NO: 2;
   - a CDR3 comprising or consisting of the amino acid sequence of SEQ ID NO: 8; and
b. a light chain variable domain (VL) comprising:
   - CDR1 comprising or consisting of the amino acid sequence of SEQ ID NO: 35;
   - CDR2 comprising or consisting of the amino acid sequence of SEQ ID NO: 36; and
   - CDR3 comprising or consisting of the amino acid sequence of SEQ ID NO: 46;

or said isolated antibody or antigen binding fragment thereof comprises:
   a. a heavy chain variable domain (VH) comprising:
      - CDR1 comprising or consisting of the amino acid sequence of SEQ ID NO: 1;
      - CDR2 comprising or consisting of the amino acid sequence of SEQ ID NO: 2;
      - CDR3 comprising or consisting of the amino acid sequence of SEQ ID NO: 4; and
   b. a light chain variable domain (VL) comprising:
      - CDR1 comprising or consisting of the amino acid sequence of SEQ ID NO: 38;
      - CDR2 comprising or consisting of the amino acid sequence of SEQ ID NO: 39; and
      - CDR3 comprising or consisting of the amino acid sequence of SEQ ID NO: 40;
or said isolated antibody or antigen binding fragment thereof comprises:
   a. a heavy chain variable domain (VH) comprising:
      - CDR1 comprising or consisting of the amino acid sequence of SEQ ID NO: 1;
      - CDR2 comprising or consisting of the amino acid sequence of SEQ ID NO: 2;
      - CDR3 comprising or consisting of the amino acid sequence of SEQ ID NO: 3; and
   b. a light chain variable domain (VL) comprising:
      - CDR1 comprising or consisting of the amino acid sequence of SEQ ID NO: 35;
      - CDR2 comprising or consisting of the amino acid sequence of SEQ ID NO: 36;
      - CDR3 comprising or consisting of the amino acid sequence of SEQ ID NO: 37;
or said isolated antibody or antigen binding fragment thereof comprises:
   a. a heavy chain variable domain (VH) comprising:
      - CDR1 comprising or consisting of the amino acid sequence of SEQ ID NO: 14;
      - CDR2 comprising or consisting of the amino acid sequence of SEQ ID NO: 15;
      - CDR3 comprising or consisting of the amino acid sequence of SEQ ID NO: 16; and
   b. a light chain variable domain (VL) comprising:
      - CDR1 comprising or consisting of the amino acid sequence of SEQ ID NO: 38;
      - CDR2 comprising or consisting of the amino acid sequence of SEQ ID NO: 39; and
      - CDR3 comprising or consisting of the amino acid sequence of SEQ ID NO: 56;
or said isolated antibody or antigen binding fragment thereof comprises:
   a. a heavy chain variable domain (VH) comprising:
      - CDR1 comprising or consisting of the amino acid sequence of SEQ ID NO: 29;
      - CDR2 comprising or consisting of the amino acid sequence of SEQ ID NO: 30;
      - CDR3 comprising or consisting of the amino acid sequence of SEQ ID NO: 31; and
   b. a light chain variable domain (VL) comprising:
      - CDR1 comprising or consisting of the amino acid sequence of SEQ ID NO: 41;
      - CDR2 comprising or consisting of the amino acid sequence of SEQ ID NO: 42; and
      - CDR3 comprising or consisting of the amino acid sequence of SEQ ID NO: 69;
or said isolated antibody or antigen binding fragment thereof comprises:
   a. a heavy chain variable domain (VH) comprising::
      - CDR1 comprising or consisting of the amino acid sequence of SEQ ID NO: 32;
      - CDR2 comprising or consisting of the amino acid sequence of SEQ ID NO: 33;
      - CDR3 comprising or consisting of the amino acid sequence of SEQ ID NO: 34; and
   b. a light chain variable domain (VL) comprising:
      - CDR1 comprising or consisting of the amino acid sequence of SEQ ID NO: 70;
      - CDR2 comprising or consisting of the amino acid sequence of SEQ ID NO: 71; and
      - CDR3 comprising or consisting of the amino acid sequence of SEQ ID NO: 72;
wherein each CDR is defined according to Kabat.

Still preferably, the isolated antibody or antigen binding fragment thereof comprising a heavy chain variable domain encoded by a nucleotide sequence selected from the group consisting of: SEQ ID NO: 109, SEQ ID NO: 110, SEQ ID NO: 111, SEQ ID NO: 112, SEQ ID NO: 113 and SEQ ID NO: 114; and/or a light chain variable domain encoded by a nucleotide sequence selected from the group consisting of: SEQ ID NO: 117, SEQ ID NO: 118, SEQ ID NO: 119, SEQ ID NO: 120, SEQ ID NO: 121 and SEQ ID NO: 122.

Still preferably, the isolated antibody or antigen binding fragment thereof is selected from the group consisting of: YU1425-A10, YU1425-A09, YU1425-E10, YU1425-C09, YU1425-C01 and

YU1025-E01 as defined in Tables 1-7, preferably it is the antibody YU1425-A10 or YU1425-A09 or YU1425-E10, or an isolated antibody or antigen binding fragment thereof that:
(a) binds specifically to an epitope on IGFBP7, said epitope being the same or similar epitope as the epitope recognized by the monoclonal antibody YU1425-E10, YU1425-A09, YU1425-A10, YU1425-C09, YU1425-C01 or YU1025-E01 as defined in Tables 1-7; or
(b) cross-competes for binding with the monoclonal antibody YU1425-E10, YU1425-A09, YU1425-A10, YU1425-C09, YU1425-C01 orYU1025-E01 as defined in Tables 1-7; or
(c) shows the same or similar binding affinity or specificity, or both, as any of monoclonal antibodies YU1425-E10, YU1425-A09, YU1425-A10, YU1425-C09, YU1425-C01 or YU1025-E01 as defined in Tables 1-7; or
(d) has one or more biological properties of an antibody molecule chosen from YU1425-E10, YU1425-A09, YU1425-A10, YU1425-C09, YU1425-C01 or YU1025-E01 as defined in Tables 1-7; and/or
(e) has one or more pharmacokinetic properties of an antibody molecule chosen from YU1425-E10, YU1425-A09, YU1425-A10, YU1425-C09, YU1425-C01 or YU 1025-E01 as defined in Tables 1-7.

Yet preferably, the isolated antibody is a human or a humanized antibody and/or being an IgG1 antibody, preferably an IgG1 kappa antibody, an IgG1 lambda antibody.

More preferably, the isolated antibody comprises a heavy chain constant region comprising or consisting of a sequence with SEQ ID NO. 125 or having at least 80%, or 85 %, or 90%, or 95%, or 100% identity to SEQ ID No. 125 and/or a light chain constant region comprising or consisting of a sequence with SEQ ID NO. 126, or having at least 80%, or 85 %, or 90%, or 95%, or 100% identity to SEQ ID No. 126 preferably wherein said heavy chain constant region is a human IgG1.

Preferably, the isolated antibody or antigen binding fragment thereof comprises:
a. a heavy chain variable domain sequence of the amino acid sequence selected from the group consisting of: SEQ ID NO: 78, 74 and 73 or
b. a light chain variable domain sequence of the amino acid sequence selected from the group consisting of: SEQ ID NO: 96, 92 and 91 or
c. the heavy chain variable domain of (a) and the light chain variable domain of (b).

More preferably, it comprises:
a heavy chain variable domain comprising or consisting of a sequence of SEQ ID NO: 78 and a light chain variable domain comprising or consisting of a sequence of SEQ ID NO: 96;
a heavy chain variable domain comprising or consisting of a sequence of SEQ ID NO: 74 and a light chain variable domain comprising or consisting of a sequence of SEQ ID NO: 92;
a heavy chain variable domain comprising or consisting of a sequence of SEQ ID NO: 73 and a light chain variable domain comprising or consisting of a sequence of SEQ ID NO: 91.

It also an object of the invention an isolated polynucleotide comprising at least one sequence that encodes the antibody or antigen binding fragment thereof, preferably said polynucleotide is a cDNA. Preferably such polynucleotide comprising or consisting of one or more nucleotide sequences selected from the group consisting of SEQ ID NO: 109 to SEQ ID NO: 114 and SEQ ID NO: 117 to SEQ ID NO: 122.

It is further object of the invention a vector comprising the isolated polynucleotide. Preferably, said vector is selected from the group consisting of a plasmid, a viral vector, a non-episomal mammalian vector, an expression vector and a recombinant expression vector, or an isolated cell comprising said isolated or said vector, preferably said isolated cell being a hybridoma or a Human Embryonic Kidney cell (HEK293).

It is further object of the invention a pharmaceutical composition comprising the antibody or antigen binding fragment thereof or the polynucleotide or the vector or the isolated cell and at least one pharmaceutically acceptable carrier, preferably said composition further comprising a second therapeutic agent.

It is also herein disclosed the antibody or antigen binding fragment thereof or the polynucleotide or the vector or the isolated cell or the pharmaceutical composition for use as a medicament.

Additionally, it is also herein disclosed the antibody or antigen binding fragment thereof or the polynucleotide or the vector or the isolated cell or the pharmaceutical composition for use in the treatment and/or prevention of vascular stress-mediated senescence, inflammation, and fibrosis or fibrotic condition, and acute stress cellular damage, preferably wherein said vascular stress-mediated senescence, inflammation, and fibrosis or fibrotic condition is selected from psoriasis, heart failure or vasculopathy; and wherein said acute stress cellular damage is acute kidney injury.

The foregoing and other features and advantages will become more apparent from the following detailed description of several embodiments, which proceeds with reference to the accompanying figures.

### BRIEF DESCRIPTION OF THE DRAWINGS

**Figure 1****.** Recombinant human IGFBP7 (rhlGFBP7) mediates apoptosis and cell death in kidney tubular cells. **A.** CASP3/7 apoptosis. **B.** Cell death. N=3 independent experiments. *p<0.05, **p<0.01, ***p<0.001.
**Figure 2****.** rhlGFBP7 mediates apoptosis and cell death in cardiomyocytes. **A.** CASP3/7 apoptosis. **B.** Cell death. N=3 independent experiments. *p<0.05, **p<0.01, ***p<0.001.
**Figure 3****.** Schematic representation of strategy for screening, generation and production of anti-IGFBP7 monoclonal antibodies (mAbs).
**Figure 4****.** Binding affinity of anti-IGFBP7 mAbs to IGFBP7 (murine, human and Streptavidin-conjugated (Strept) human).
**Figure 5****.** In vitro validation of binding of anti-IGFBP7 mAbs to human IGFBP7 as compared to commercial monoclonal (mAb; ab171085 AbCam) and polyclonal (pAb; AF1334 R&D Systems) antibodies.
**Figure 6****.** Anti-IGFBP7 mAbs YU1425-A10, YU1425-A09, YU1425-C01 and YU1425-C09 protect tubular cells from death in presence of IGFBP7. YU1425-A10, A10; YU1425-A09, A09; YU1425-C01, C01; YU1425-C09, C09.
**Figure 7****.** Anti-IGFBP7 mAbs YU1425-A10, YU1425-E10, YU1425-E01 protect endothelial cells from death in presence of IGFBP7. YU1425-A10, A10; YU1425-E10, E10; YU1425-E01, E01.

### DETAILED DESCRIPTION OF THE INVENTION

Unless otherwise noted, technical terms are used according to conventional usage in the art.

The terms "comprising", "comprises" and "comprised of" as used herein are synonymous with "including" or "includes" or "containing" or "contains" and are inclusive or open-ended and do not exclude additional, non-recited members, elements or steps. The terms "comprising", "comprises" and "comprised of" also include the term "consisting of".

The terms "percent sequence identity" (% sequence identity), "percent identical" (% identical) and the like refer to percent sequence identity between two nucleotide sequences or between two amino acid sequences calculated by aligning the two sequences, determining the number of matches of nucleotides or amino acid residues between the two sequences, dividing the number of matches by the length of the aligned region (i.e., the number of aligned nucleotides or amino acid residues), and multiplying by 100 to arrive at a percent sequence identity value. For calculation of the percent sequence identity (% sequence identity), unless otherwise specified, two or more sequences are aligned using the EMBOSS Needle Pairwise Sequence Alignment software tool based on the Needleman and Wunsch algorithm (available at www.ebi.ac.uk/Tools/psa/emboss_needle) with the following parameters: Matrix: BLOSUM62 (for protein sequences) or DNAfull (for DNA sequences); Gap Open: 10; Gap Extend: 0.5; End Gap Penalty: false; End Gap Open: 10; and End Gap Extend: 0.5.

Additional methods of alignment of nucleotide and amino acid sequences for comparison are also well known in the art. The local homology algorithm (BESTFIT) of Smith and Waterman (1981) Adv. Appl. Math 2:482, may permit optimal alignment of compared sequences; by the homology alignment algorithm (GAP) of Needleman and Wunsch (1970) J. Mol. Biol. 48:443-453; by the search for similarity method (Tfasta and Fasta) of Pearson and Lipman (1988) Proc. Natl. Acad. Sci. USA 85:2444; by computerized implementations of these algorithms, including, but not limited to: CLUSTAL in the PC/Gene program by Intelligenetics, Mountain View, Calif., GAP, BESTFIT, BLAST, FASTA and TFASTA in the Wisconsin Genetics Software Package, Version 8 (available from Genetics Computer Group (GCG^{™} programs (Accelrys, Inc., San Diego, Calif.)). The CLUSTAL program is well described by Higgins and Sharp (1988) Gene 73:237-244; Higgins and Sharp (1989) CABIOS 5: 151-153; Corpet, et al. (1988) Nucleic Acids Res. 16: 10881-10890; Huang, et al. (1992) Computer Applications in the Biosciences 8: 155-165; and Pearson, et al. (1994) Meth. Mol. Biol. 24:307-331. An example of a good program to use for optimal global alignment of multiple sequences is PileUp (Feng and Doolittle (1987) J. Mol. Evol. 25:351- 260, which is similar to the method described by Higgins and Sharp (1989) CABIOS 5: 151- 153 (and is hereby incorporated by reference). The BLAST family of programs that can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences. See, Current Protocols in Molecular Biology, Chapter 19, Ausubel, etal., eds., Greene Publishing and Wiley-Interscience, New York (1995). An updated version of the BLAST family of programs includes the BLAST+ suite. (Camacho, C., et al. (2009 Dec 15) BLAST+: architecture and applications. BMC Bioinformatics 10:421).

In particular, in the present invention "at least 80 % identity" means that the identity may be at least 80%, or 85 %, or 90%, or 95%, or 100% sequence identity to referred sequences wherein the % of sequence identity is calculated as described above. This applies to all the mentioned % of identity. In the present invention "at least 95% identity" means that the identity may be at least 95%, 96%, 97%, 98%, 99%, or 100% sequence identity to referred sequences. This applies to all the mentioned % of identity. In the present invention "at least 98% identity" means that the identity may be at least 98%, 99%, or 100% sequence identity to referred sequences. This applies to all the mentioned % of identity. Preferably, the % of identity relates to the full length of the referred sequence.

The antibodies of the invention specifically bind to human and/or mouse IGFBP7. Preferably, to human IGFBP7.

As discussed herein, these antibodies are collectively referred to as "anti-IGFBP7 antibodies". All of such antibodies are encompassed by the discussion herein. The respective antibodies can be used alone or in combination in the methods of the invention.

By "antibodies that specifically bind" IGFBP7 is intended that the antibodies will not substantially cross react with another, non-homologous, human polypeptide. By "not substantially cross react" is intended that the antibody or fragment has a binding affinity for a non-homologous protein which is less than 10%, more preferably less than 5%, and even more preferably less than 1%, of the binding affinity for IGFBP7.

In various embodiments, an antibody that "specifically binds" IGFBP7, as used herein, includes antibodies that bind human IGFBP7 with a KD of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or about 0.5 nM or about 0.05 nM, as measured with an Octet biolayer interferometry device or in a surface plasmon resonance assay, for example using the BIAcore^{™} system (Biacore Life Sciences division of GE Healthcare, Piscataway, NJ) or kinetic exclusion assays or binding to IGFBP7 receptor expressed by HEK293 cells or any known method in the art.

The term "antibody" herein is used in the broadest sense understood in the art, including but not limited to, all polypeptides described as antibodies in Sumit G, Wei W, Tsutomu and Satoshi O, Antibodies 2013; 2: 452-500, incorporated herein by reference and including, but not limited to, all formats described in Martin et al., MAbs 15(1):2191301 (2023).

For example, the term "antibody", as used herein encompasses monoclonal antibodies, polyclonal antibodies, monospecific and multi-specific antibodies (e.g., bispecific antibodies), and antibody fragments so long as the fragment exhibits the desired antigen-binding activity (antigen-binding fragments).

The terms "antigen-binding fragment" of an antibody or equivalently "antigen-binding portion" of an antibody and the like, as used herein, include any naturally occurring, enzymatically obtainable, synthetic, or genetically engineered polypeptide or glycoprotein that comprises a portion of an antibody and that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

As with full antibody molecules, antigen-binding fragments may be monospecific or multi-specific (e.g., bispecific). A multi-specific antigen-binding fragment of an antibody will typically comprise at least two different antigen binding moieties, wherein each antigen binding moiety is capable of specifically binding to a separate antigen or to a different epitope on the same antigen.

In particular embodiments, an antigen-binding fragment of an antibody comprises at least one variable domain covalently linked to at least one constant domain. Non-limiting, exemplary configurations of variable and constant domains that may be found within an antigen-binding fragment of an antibody include: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may in various embodiments consist of at least 2 (e.g., 5, 10, 15, 20, 40, 60 or more) amino acids which result in a flexible or semi-flexible linkage between adjacent variable and/or constant domains in a single polypeptide molecule. Moreover, an antigen-binding fragment of an antibody may in various embodiments comprise a homo-dimer or hetero-dimer (or other multimer) of any of the variable and constant domain configurations listed above in non-covalent association with one another and/or with one or more monomeric VH or VL domain (e.g., by disulfide bond(s)).

The term "antigen-binding fragment" of an antibody further includes single domain antibodies. A single-domain antibody is an antibody fragment consisting of a single monomeric variable antibody domain. In some embodiments, the single-domain antibody is derived from the variable domain of the antibody heavy chain from camelids (also termed nanobodies, or VHH fragments). In some embodiments, the single-domain antibody is an autonomous human heavy chain variable domain (aVH) or VNAR fragments derived from sharks.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain- deleted antibodies, chimeric antibodies, CDR-grafted antibodies, diabodies, triabodies, tetrabodies, minibodies, nanobodies (e.g., monovalent nanobodies, and bivalent nanobodies), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a VH domain associated with a VL domain, the VH and VL domains may be situated relative to one another in any suitable arrangement. For example, the variable region may be dimeric and contain VH-VH, VH-VL or VL-VL dimers. Alternatively, the antigen-binding fragment of an antibody may contain a monomeric VH or VL domain.

The term "antibody," as used herein, also includes ADC (antibody drug conjugate) and payload fusion antibodies.

As used herein, the term "antigen binding molecule" refers in its broadest sense to a molecule that specifically binds an antigenic determinant. Examples of antigen binding molecules are antibodies, including antigen-binding antibody fragments, and scaffold antigen binding proteins. The term "antigen binding moiety" refers to the portion of an antigen binding molecule that specifically binds to an antigenic determinant. Antigen binding moieties include antibodies and antigen-binding fragments thereof, such as scFv, that are capable of specific binding to an antigen on a target cell. In a particular aspect, the antigen binding moiety is able to direct the entity to which it is attached, such as a cell, to a target site.

In addition, antigen binding moieties capable of specific binding to a target cell antigen include scaffold antigen binding proteins as defined herein below, e.g. binding domains which are based on designed repeat proteins or designed repeat domains such as designed ankyrin repeat proteins (DARPins) (see e.g. WO 2002/020565) or Lipocalins (Anticalin). Designed Ankyrin Repeat Proteins (DARPins) are derived from Ankyrin, which is a family of proteins that mediate attachment of integral membrane proteins to the cytoskeleton. A single ankyrin repeat is a 33-residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028.

In certain embodiments, antibodies and antigen binding molecules provided herein are altered to increase or decrease the extent to which the antigen binding moiety is glycosylated. Glycosylation variants of the molecules may be conveniently obtained by altering the amino acid sequence such that one or more glycosylation sites is created or removed. Where the antigen binding molecule comprises an Fc region, the carbohydrate attached thereto may be altered. In one aspect, variants of antigen binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of antigen binding molecules of the invention include those with bisected oligosaccharides, e.g., in which a biantennary oligosaccharide attached to the Fc region is bisected by GlcNAc. Such variants may have reduced fucosylation and/or improved ADCC function, see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.) and WO 1999/22764 (Raju, S.).

In certain embodiments, it may be desirable to create cysteine engineered variants of the antibody or antigen binding molecule of the invention, e.g., "thioMAbs," in which one or more residues of the molecule are substituted with cysteine residues. In particular embodiments, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the antibody or antigen binding molecules provided herein may be further modified to contain additional non-proteinaceous moieties that are known in the art and readily available. The moieties suitable for derivatization of the antibody or antigen binding molecule include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-I,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another aspect, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed. In another aspect, immunoconjugates of the antigen binding molecules provided herein may be obtained. An "immunoconjugate" is an antibody conjugated to one or more heterologous molecule(s), including but not limited to a cytotoxic agent.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity, or not. In certain embodiments, the constant region is an IgG1, IgG2, IgG3, IgG4 constant region.

The instant invention encompasses in various embodiments antibodies having one or more mutations in the hinge, CH2 or CH3 region which may be desirable, for example, in production, to improve the yield of the desired antibody form. In some embodiments, for example, the antibodies described herein comprise a human IgG1 constant region.

In certain embodiments, the antibody comprises one or more mutations in the constant region that increase serum half-life, including those described in US Patent Nos. 7,083,784, 8,323,962 and Dall'Aqua et al., J. Biol. Chem. 281(33):23514-23524 (2006); Hinton et al., J. Immunology 176:346-356 (2006); Yeung et al., J. Immunology 182:7663-7671 (2009); and Petkova et al., Intn'l Immunology,18: 1759-1769 (2006), incorporated herein by reference in their entireties.

In a preferred embodiment, the antibody molecule includes a heavy chain constant region for an IgG1, e.g., a human IgG1. In one embodiment, the human IgG1 includes a substitution at position 297 (e.g., an Asn to Ala substitution). In one embodiment the human IgG1 includes a substitution at position 250, a substitution at position 428, or both (e.g., a Thr to Gln substitution at position 250 and/or a Met to Leu substitution at position 428). In one embodiment, the human IgG1 includes a substitution at position 234, a substitution at position 235, or both (e.g., a Leu to Ala substitution at position 234 and/or a Leu to Ala substitution at position 235). In preferred embodiments, the antibody includes a heavy chain constant region and/or a light chain constant region having one of the amino acid sequences of Table 7.

The term "human antibody", as used herein, is intended to include antibodies having variable and constant regions derived from human germline immunoglobulin sequences. The human antibodies featured in the invention may in various embodiments nonetheless include amino acid residues not encoded by human germline immunoglobulin sequences (e.g., mutations introduced by random or site- specific mutagenesis in vitro or by somatic mutation in vivo), for example in the CDRs and, in some embodiments, CDR3. However, the term "human antibody", as used herein, is not intended to include antibodies in which CDR sequences are derived from the germline of another mammalian species, such as a mouse, which have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody." In various embodiments, the isolated antibody also includes an antibody in situ within a recombinant cell. In other embodiments, isolated antibodies are antibodies that have been subjected to at least one purification or isolation step. In various embodiments, an isolated antibody may be substantially free of other cellular material and/or chemicals.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

In an embodiment, the antibody molecule includes a heavy chain variable domain and a constant region, a light chain variable domain and a constant region, or both, comprising the amino acid sequence of any of YU1425-A10, YU1425-A09, YU1425-E10, YU1425-C09, YU1425-C01 and YU1425-E01 as defined in Tables 1-4, and 7 or encoded by a nucleotide sequence in Tables 5-6; or a sequence substantially identical (e.g., at least 80%, 85%, 90%, 92%, 95%, 97%, 98%, 99% or higher identical) to any of the aforesaid sequences. The antibody molecule, optionally, comprises a leader sequence from a heavy chain, a light chain, or both.

In yet another embodiment, the antibody molecule includes at least one, two, or three complementarity determining regions (CDRs) from a heavy chain variable region of an antibody described herein, e.g., an antibody chosen from any ofYU1425-A10, YU1425-A09, YU1425-E10, YU1425-C09, YU1425-C01 and YU1425-E01 as defined in Tables 1-4, and 7 or encoded by a nucleotide sequence in Tables 5-6; or a sequence substantially identical (e.g., at least 80%, 85%, 90%, 92%, 95%, 97%, 98%, 99% or higher identical) to any of the aforesaid sequences.

In yet another embodiment, the antibody molecule includes at least one, two, or three CDRs (or collectively all of the CDRs) from a heavy chain variable region comprising an amino acid sequence shown in Tables 1-2 or encoded by a nucleotide sequence shown in Tables 5-6. In one embodiment, one or more of the CDRs (or collectively all of the CDRs) have one, two, three, four, five, six or more changes, e.g., amino acid substitutions or deletions, relative to the amino acid sequence shown in Tables 3 or encoded by a nucleotide sequence shown in Table 5. In certain embodiments, the antibody molecule includes a substitution in a heavy chain CDR, e.g., one or more substitutions in a CDR1, CDR2 and/or CDR3 of the heavy chain.

In yet another embodiment, the antibody molecule includes at least one, two, or three CDRs from a light chain variable region of an antibody described herein, e.g., an antibody chosen from any of any of as defined in Tables 4, or encoded by the nucleotide sequence in Table 6; or a sequence substantially identical (e.g., at least 80%, 85%, 90%, 92%, 95%, 97%, 98%, 99% or higher identical) to any of the aforesaid sequence. In certain embodiments, the antibody molecule includes a substitution in a light chain CDR, e.g., one or more substitutions in a CDR1, CDR2 and/or CDR3 of the light chain.

In another embodiment, the antibody molecule includes at least one, two, three, four, five or six CDRs (or collectively all of the CDRs) from a heavy and light chain variable region comprising an amino acid sequence shown in Tables 1-4 or encoded by a nucleotide sequence shown in Tables 5-6. In one embodiment, one or more of the CDRs (or collectively all the CDRs) have one, two, three, four, five, six or more changes, e.g., amino acid substitutions or deletions, relative to the amino acid sequence shown in Tables 1-4 or encoded by a nucleotide sequence shown in Tables 5-6.

In one embodiment, the antibody molecule includes all six CDRs from an antibody described herein, e.g., an antibody chosen from any of YU1425-A10, YU1425-A09, YU1425-E10, YU1425-C09, YU1425-C01 and YU1425-E01 as defined in Tables 1-4 or encoded by the nucleotide sequence in Tables 5-6 or closely related CDRs, e.g., CDRs which are identical or which have at least one amino acid alteration, but not more than two, three or four alterations (e.g., substitutions, deletions, or insertions, e.g., conservative substitutions). In one embodiment, the antibody molecule may include any CDR described herein.

In one embodiment, one or more of the CDRs (or collectively all of the CDRs) have one, two, three, four, five, six or more changes, e.g., amino acid substitutions or deletions, relative to the amino acid sequence shown in Tables 1-4, in particular in Tables 1-2.

In certain embodiments, the antibody includes a substitution in a light chain CDR, e.g., one or more substitutions in a CDR1, CDR2 and/or CDR3 of the light chain.

In certain embodiments, the antibody includes a substitution in a heavy chain CDR, e.g., one or more substitutions in a CDR1, CDR2 and/or CDR3 of the heavy chain.

In certain embodiments, the antibody includes from 1 to 5 amino acid substitutions among the six CDRs, i.e. CDR1, CDR2, CDR3 of the heavy chain, CDR1, CDR2 and CDR3 of the light chain.

Preferably, the term "CDR" is a CDR as defined by Kabat, based on sequence comparisons. CDRH1, CDRH2 and CDRH3 denote the heavy chain CDRs, and CDRL1, CDRL2 and CDRL3 denote the light chain CDRs.

In an embodiment, the antibody molecule includes at least one, two, or three CDRs according to Kabat et al., e.g., as set out in any one of Tables 1-2, from a heavy chain variable region of an antibody described herein, or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to one, two, or three CDRs according to Kabat et al. shown in Tables 1-2.

In an embodiment, the antibody molecule includes at least one, two, or three CDRs according to Kabat et al., e.g., as set out in any one of Tables 1-2, from a light chain variable region of an antibody described herein, or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to one, two, or three CDRs according to Kabat et al. shown in Tables 1-2.

In yet another embodiment, the antibody molecule includes all six CDRs according to Kabat et al., e.g., all six CDRs according to the Kabat definition as set out in Tables 1-2, from the heavy and light chain variable regions of an antibody described herein, e.g., an antibody chosen from any of YU1425-A10, YU1425-A09, YU1425-E10, YU1425-C09, YU1425-C01 and YU1425-E01 as defined in Tables 1-4 or encoded by the nucleotide sequence in Tables 5-6; or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to all six CDRs according to Kabat definition. In one embodiment, the antibody molecule may include any CDR described herein.

In another embodiment, the antibody includes at least one, two, or three CDRs of a heavy chain variable region according to Chothia, AbM, Contact or IMGT definition; or which have at least one amino acid alteration, but not more than two, three or four alterations (e.g., substitutions, deletions, or insertions, e.g., conservative substitutions).

In another embodiment, the antibody includes at least one, two, or three CDRs of a light chain variable region according to Chothia, AbM, Contact or IMGT definition; or which have at least one amino acid alteration, but not more than two, three or four alterations (e.g., substitutions, deletions, or insertions, e.g., conservative substitutions).

In certain embodiments, the antibody molecule includes a combination of CDRs defined according to the Kabat, Chothia, AbM, Contact or IMGT definition.

Preferred antibodies are antibodies YU1425-A10, YU1425-A09, YU1425-E10, YU1425-C09, YU1425-C01 and YU1425-E01 as defined in Tables 1-4, 7 or encoded by the nucleotide sequence in Tables 5-6.

In one embodiment, the antibody includes:
(i) a heavy chain variable region (VH) including a VHCDR1 amino acid sequence chosen from any one of SEQ ID NO: 1, 14, 29, and 32; a VHCDR2 amino acid sequence chosen from any one of SEQ ID NO: 2, 15, 30 and 33; and a VHCDR3 amino acid sequence chosen from any one of SEQ ID NO: 8, 4, 3, 16, 31, and 34; and
(ii) a light chain variable region (VL) including a VLCDR1 amino acid sequence chosen from any one of SEQ ID NO: 35, 38, 41 and 70, a VLCDR2 amino acid sequence chosen from any one of SEQ ID NO: 36, 39, 42 and 71, and a VLCDR3 amino acid sequence chosen from SEQ ID NO: 46, 40, 37, 56, 69 and 72.

In one embodiment, the light or the heavy chain variable framework (e.g., the region encompassing at least FR1, FR2, FR3, and optionally FR4) of the antibody molecule can be chosen from: (a) a light or heavy chain variable framework including at least 80%, 85%, 87% 90%, 92%, 93%, 95%, 97%, 98%, or preferably 100% of the amino acid residues from a human light or heavy chain variable framework, e.g., a light or heavy chain variable framework residue from a human mature antibody, a human germline sequence, or a human consensus sequence; (b) a light or heavy chain variable framework including from 20% to 80%, 40% to 60%, 60% to 90%, or 70% to 95% of the amino acid residues from a human light or heavy chain variable framework, e.g., a light or heavy chain variable framework residue from a human mature antibody, a human germline sequence, or a human consensus sequence; (c) a non-human framework (e.g., a rodent framework); or (d) a non-human framework that has been modified, e.g., to remove antigenic or cytotoxic determinants, e.g., deimmunized, or partially humanized. In one embodiment, the light or heavy chain variable framework region (particularly FR1, FR2 and/or FR3) includes a light or heavy chain variable framework sequence at least 70, 75, 80, 85, 87, 88, 90, 92, 94, 95, 96, 97, 98, 99% identical or identical to the frameworks of a VL or VH segment of a human germline gene.

In an embodiment, the antibody molecule includes one or more heavy chain framework region (e.g., any of VHFW1 (type a), VHFW1 (type b), VHFW1 (type c), VHFW1 (type d), VHFW2 (type a), VHFW2 (type a'), VHFW2 (type b), VHFW2 (type c), VHFW2 (type d), VHFW2 (type e), VHFW3 (type a), VHFW3 (type b), VHFW3 (type c), VHFW3 (type d), VHFW3 (type e), or VHFW4, or any combination thereof, e.g., a framework combination for any of YU1425-A10, YU1425-A09, YU1425-E10, YU1425-C09, YU1425-C01 and YU1425-E01, or a sequence substantially identical thereto (e.g., a sequence at least about 85%, 90%, 95%, 99% or more identical thereto).

In another embodiment, the antibody molecule includes one or more light chain framework region (e.g., any of VLFW1 (type a), VLFW1 (type b), VLFW1 (type c), VLFW1 (type d), VLFW1 (type e), VLFW1 (type f), VLFW2 (type a), VLFW2 (type c), VLFW3 (type a), VLFW3 (type b), VLFW3 (type c), VLFW3 (type d), VLFW3 (type e), VLFW3 (type f), VLFW3 (type g), or VLFW4, or any combination thereof, e.g., a framework combination for any of YU1425-A10, YU1425-A09, YU1425-E10, YU1425-C09, YU1425-C01 and YU1425-E01, or a sequence substantially identical thereto (e.g., a sequence at least about 85%, 90%, 95%, 99% or more identical thereto).

In another embodiment, the antibody molecule includes one or more heavy chain framework regions and one or more light chain framework regions as described herein.

In certain embodiments, the antibody molecule comprises a heavy chain variable domain having at least one, two, three, four, five, six, seven, ten, fifteen, twenty or more changes, e.g., amino acid substitutions or deletions with respect to the sequence of SEQ ID NO: 78, SEQ ID NO: 74, SEQ ID NO: 73, SEQ ID NO: 82, SEQ ID NO: 89 or SEQ ID NO: 90.

In certain embodiments, the antibody molecule comprises a light chain variable domain having at least one, two, three, four, five, six, seven, ten, fifteen, twenty or more changes, e.g., amino acid substitutions or deletions with respect to the sequence of SEQ ID NO: 96, SEQ ID NO: 92, SEQ ID NO: 91, SEQ ID NO: 100, SEQ ID NO: 107 or SEQ ID NO: 108.

In one embodiment, the heavy or light chain variable region, or both, of the antibody molecule includes an amino acid sequence encoded by a nucleic acid sequence described herein or a nucleic acid that hybridizes to a nucleic acid sequence described herein, in particular a nucleic acid sequence as shown in Tables 5 and 6 or its complement, e.g., under low stringency, medium stringency, or high stringency, or other hybridization condition described herein.

In another embodiment, the antibody molecule comprises at least one, two, three, or four antigen-binding regions, e.g., variable regions, having an amino acid sequence as set forth in anyone of Tables 1-4 or a sequence substantially identical thereto (e.g., a sequence at least about 85%, 90%, 95%, 99% or more identical thereto, or which differs by no more than 1, 2, 5, 10, or 15 amino acid residues from the sequences shown in Tables 1-4).

In another embodiment, the antibody molecule includes a VH and/or VL domain encoded by a nucleic acid having a nucleotide sequence as set forth in Tables 5 and 6 or a sequence substantially identical thereto (e.g., a sequence at least about 85%, 90%, 95%, 99% or more identical thereto, or which differs by no more than 3, 6, 15, 30, or 45 nucleotides from the sequences shown in Tables 5-6).

In yet other embodiments, the antibody molecule has a heavy chain constant region (Fc) chosen from, e.g., the heavy chain constant regions of IgG1, IgG2, IgG3, IgG4, IgM, !gA1, IgA2, IgD, and IgE; particularly, chosen from, e.g., the heavy chain constant regions of IgG1, IgG2, IgG3, and IgG4, more particularly, the heavy chain constant region of IgG1 (e.g., human IgG1, IgG2 or IgG4). In one embodiment, the heavy chain constant region is human IgG1. In another embodiment, the antibody molecule has a light chain constant region chosen from, e.g., the light chain constant regions of kappa or lambda. In one embodiment, the constant region is altered, e.g., mutated, to modify the properties of the antibody (e.g., to increase or decrease one or more of: Fc receptor binding, antibody glycosylation, the number of cysteine residues, effector cell function, complement function, half-life, aggregation, and stability). In a preferred embodiment, the antibody has a heavy chain constant region comprising or consisting of a sequence with SEQ ID NO. 125. In a preferred embodiment, the antibody has a light chain constant region comprising or consisting of a sequence with SEQ ID NO. 126.

In one embodiment, the antibody is isolated or recombinant.

In one embodiment, the antibody is a humanized or human antibody molecule.

The antibody of the invention may have framework sequences from any species. Preferably, it may have a mouse or human framework. As used herein the term "framework (FR) amino acid residues" refers to those amino acids in the framework region of an immunoglobulin chain. The term "framework region" or "FR region" as used herein, includes the amino acid residues that are part of the variable region, but are not part of the CDRs (e.g., using the Kabat definition of CDRs). A monoclonal antibody comprising the CDRs sequences of any of the antibody herein described is also within the scope of the invention.

Methods for producing a monoclonal antibody with the CDR sequences as mentioned above are known in the art and include the introduction of the nucleic acid sequences encoding the CDRs into suitable expression vectors encoding the desired framework sequences.

The anti-IGFBP7 antibodies described herein and useful for the methods featured herein may in various embodiments include one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases.

The present invention includes in various embodiments antibodies and methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations").

Numerous antibodies and antigen- binding fragments may be constructed which comprise one or more individual germline mutations or combinations thereof. In certain embodiments, all of the framework and/or CDR residues within the VH and/or VL domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (i.e., a germline sequence that is different from the germline sequence from which the antibody was originally derived). Furthermore, the antibodies may contain any combination of two or more germline mutations within the framework and/or CDR regions, e.g., wherein certain individual residues are mutated to the corresponding residue of a certain germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The antibody according to the invention may form a homo- or heterodimer or a homo- or heteromultimer, whereby "dimer" and "multimer" means that two and at least three antibodies, respectively, may combine to form a complex. The prefix "homo" means that a complex may be formed of identical antibody molecules, whereby the prefix "hetero" means that a complex may be formed of different antibody molecules. In general, the term "antibody" is intended to comprise all above-mentioned immunoglobulin isotypes, i.e. the antibody may be an IgA, IgD, IgE, IgG or IgM antibody, including any subclass of these isotypes. Preferably, the antibody is an IgG antibody, more preferred the antibody is an IgG1 antibody. Since the antibody may be expressed and produced recombinantly, the antibody may also comprise two different constant regions of heavy chains, e.g. one IgG1 and one IgG2 heavy chain, or heavy chains from different species. However, the heavy chains preferably are from the same species.

To generate whole antibodies, PCR primers including VH or VL nucleotide sequences, a restriction site, and a flanking sequence to protect the restriction site can be used to amplify the VH or VL sequences in scFv clones or other clones. Utilizing cloning techniques known to those of skill in the art, the PCR amplified VH domains can be cloned into vectors expressing a heavy chain constant region, e.g., the human gamma 4 constant region, and the PCR amplified VL domains can be cloned into vectors expressing a light chain constant region, e.g., human kappa or lambda constant regions. In certain embodiments, the vectors for expressing the VH or VL domains comprise a promoter, a secretion signal, a cloning site for the variable region, constant domains, and a selection marker such as neomycin. The VH and VL domains can also be cloned into one vector expressing the necessary constant regions. The heavy chain conversion vectors and light chain conversion vectors are then co- transfected into cell lines to generate stable or transient cell lines that express full-length antibodies, e.g., IgG, using techniques known to those of skill in the art.

The invention also features a nucleic acid molecule that comprises one or both nucleotide sequences that encode heavy and light chain variable regions, CDRs, framework regions of the antibody, as described herein. In certain embodiments, the nucleotide sequence that encodes the antibody molecule is codon optimized. For example, the invention features a first and second nucleic acid encoding heavy and light chain variable regions, respectively, of an antibody molecule chosen from one or more of, e.g., any YU1425-A10, YU1425-A09, YU1425-E10, YU1425-C09, YU1425-C01 and YU1425-E01 as defined in Tables 1-4, or encoded by the nucleotide sequence in Tables 5-6, or a sequence substantially identical thereto. For example, the nucleic acid can comprise a nucleotide sequence as set forth in Tables 5 and 6, or a sequence substantially identical thereto (e.g., a sequence at least about 85%, 90%, 95%, 99% or more identical thereto, or which differs by no more than 3, 6, 15, 30, or 45 nucleotides from the sequences shown in Tables 5 and 6).

In other embodiments, the nucleic acid molecule comprises a nucleotide sequence that encodes a heavy chain variable domain and/or a heavy chain constant region comprising the amino acid sequence of the heavy chain of any of antibody YU1425-A10, YU1425-A09, YU1425-E10, YU1425-C09, YU1425-C01 and YU1425-E01 as defined in Table 3 or 7 a nucleotide sequence as defined in Table 5; or a sequence substantially identical (e.g., a sequence at least about 85%, 90%, 95%, 99% or more identical) to any of the aforesaid sequences.

In other embodiments, the nucleic acid molecule comprises a nucleotide sequence that encodes a light chain variable domain and/or a light chain constant region comprising the amino acid sequence of the light chain of any of antibody YU1425-A10, YU1425-A09, YU1425-E10, YU 1425-C09, YU1425-C01 and YU1425-E01 as defined in Table 4 or 7 or a nucleotide sequence as defined in Table 6, or a sequence substantially identical (e.g., a sequence at least about 85%, 90%, 95%, 99% or more identical) to any of the aforesaid sequences.

Preferably, said nucleic acid molecule comprises a nucleotide sequence selected from the group consisting of: SEQ ID NO: 109, SEQ ID NO: 110, SEQ ID NO: 111, SEQ ID NO: 112, SEQ ID NO: 113 and SEQ ID NO: 114 encoding for a heavy chain variable domain and/or a nucleotide sequence selected from the group consisting of: SEQ ID NO: 117, SEQ ID NO: 118, SEQ ID NO: 119, SEQ ID NO: 120, SEQ ID NO: 121 and SEQ ID NO: 122 encoding for a light chain variable domain.

The aforesaid nucleotide sequences encoding the herein disclosed heavy and light chain variable domain and constant regions can be present in separate nucleic acid molecules or in the same nucleic acid molecule. In certain embodiments, the nucleic acid molecules comprise a nucleotide sequence encoding a leader sequence.

In certain embodiments, the nucleic acid molecule comprises a nucleotide sequence encoding at least one, two, or three CDRs from a heavy chain variable region having an amino acid sequence as set forth in Table 1, or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule comprises a nucleotide sequence encoding at least one, two, or three CDRs, or hypervariable loops, from a light chain variable region having an amino acid sequence as set forth in Table 2 or a sequence substantially homologous thereto (e.g., a sequence at least about 85%, 90%, 95%, 99% or more identical thereto, and/or having one, two, three or more substitutions, insertions or deletions, e.g., conserved substitutions).

In yet another embodiment, the nucleic acid molecule comprises a nucleotide sequence encoding at least one, two, three, four, five, or six CDRs from heavy and light chain variable regions having an amino acid sequence as set forth in Tables 1-2 or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule includes one or more heavy chain framework regions and one or more light chain framework regions as described herein. The heavy and light chain framework regions may be present in the same vector or separate vectors.

In another aspect, the application features host cells and vectors containing the nucleic acids described herein or modified for codon optimization according to known methods. The nucleic acids may be present in a single vector or separate vectors present in the same host cell or separate host cell. The host cell can be a eukaryotic cell, e.g., a mammalian cell, an insect cell, a yeast cell, or a prokaryotic cell, e.g., E. coli. For example, the mammalian cell can be a cultured cell or a cell line. Exemplary mammalian cells include Human Embryonic Kidney cell (HEK293), lymphocytic cell lines (e.g., NSO), Chinese hamster ovary cells (CHO), COS cells, oocyte cells, and cells from a transgenic animal, e.g., mammary epithelial cell. Preferably, it is HEK293 cell. Generally, expression vectors are plasmids which are used to introduce a desired nucleic acid sequence, such as a gene, into a target cell, resulting in the transcription and translation of the protein encoded by the nucleic acid sequence, i.e. the chimeric antigen receptor, the antibody or the binding molecule. Therefore, the expression vector in general comprises regulatory sequences, such as promoter and enhancer regions, as well as a polyadenylation site in order to direct efficient transcription of the nucleic acid sequence on the expression vector. The expression vector may further comprise additional necessary or useful regions, such as a selectable marker for selection in eukaryotic or prokaryotic cells, a purification tag for the purification of the resulting protein, a multiple cloning site or an origin of replication.

Usually, the expression vector may be a viral or a non- viral vector. In general, various kinds of viral vectors, such as retroviral vectors, e.g. lentiviral or adenoviral vectors, or plasmids may be used. In a preferred embodiment, the expression vector according to aspect five is a viral vector. In a more preferred embodiment, the expression vector is a lentiviral vector.

In one aspect, the invention features a method of providing an antibody molecule described herein. The method includes: providing a IGFBP7 antigen (e.g., an antigen comprising at least a portion of a CD248 epitope); obtaining an antibody molecule that specifically binds to the IGFBP7 polypeptide; and evaluating if the antibody molecule specifically binds to the IGFBP7 polypeptide, or evaluating efficacy of the antibody molecule in modulating, e.g., inhibiting, the activity of the IGFBP7. The method can further include administering the antibody molecule to a subject, e.g., a human or non-human animal.

In the context of the present invention for "an IGFBP7 receptor" is intended a chemical structure consisting of protein, within a cell or on the cell surface (e.g. endothelial cell, kidney tubular cell, breast cancer cell), characterized by selective binding of IGFBP7 and a corresponding physiologic effect that accompanies the binding. In an exemplary embodiment IGFBP7 receptor is a CD93 receptor or an IGF-1 receptor.

### Therapeutic Methods and Pharmaceutical Compositions

In another aspect, the invention provides compositions, e.g., pharmaceutical compositions, which include a pharmaceutically acceptable carrier, excipient or stabilizer, and at least one of the antibody molecules described herein or antigen-binding fragment thereof. In one embodiment, the composition, e.g., the pharmaceutical composition, includes a combination of the antibody molecule or antigen-binding fragment and one or more agents, e.g., a second therapeutic agent or other antibody molecule, as described herein.

Preferably, said second therapeutic agent is selected from nintedanib, pirfenidone, pamrevlumab, galectin-3 inhibitors, autotaxin inhibitors, pentraxins, tocilizumab, rituximab and romilkimab.

In one embodiment, the antibody molecule is conjugated to a label or a therapeutic agent.

The term "bioequivalent" as used herein, refers to a molecule having similar bioavailability (rate and extent of availability) after administration at the same molar dose and under similar conditions (e.g., same route of administration), such that the effect, with respect to both efficacy and safety, can be expected to be essentially same as the comparator molecule. Two pharmaceutical compositions comprising an anti- IGFBP7 antibody are bioequivalent if they are pharmaceutically equivalent, meaning they contain the same amount of active ingredient (e.g., anti- IGFBP7 antibody), in the same dosage form, for the same route of administration and meeting the same or comparable standards. Bioequivalence can be determined, for example, by an in vivo study comparing a pharmacokinetic parameter for the two compositions. Parameters commonly used in bioequivalence studies include peak plasma concentration (Cmax) and area under the plasma drug concentration time curve (AUC).

The antibody or antigen-binding fragment is administered to the subject in various embodiments in a formulation comprising suitable carriers, excipients, and other agents to provide improved transfer, delivery, tolerance, and the like, and suitable for an intravenous or subcutaneous injection.

The injectable preparations may be prepared by methods publicly known. For example, injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 20 or 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injectable preparation thus prepared can be filled in an appropriate ampoule.

The antibody or antigen-binding fragment according to the invention can be administered to the subject using any acceptable device or mechanism. For example, the administration can be accomplished using a syringe and needle or with a reusable pen and/or autoinjector delivery device. The methods of the present invention include the use of numerous reusable pens and/or autoinjector delivery devices to administer an antibody (or pharmaceutical formulation comprising the antibody). Examples of such devices include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany), to name only a few. Examples of disposable pen and/or autoinjector delivery devices having applications in subcutaneous delivery of a pharmaceutical composition of the present invention include, but are not limited to, the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), the HUMIRA^{™} Pen (Abbott Labs, Abbott Park, IL), the DAI^{®} Auto Injector (SHL Group) and any auto-injector featuring the PUSHCLICK^{™} technology (SHL Group), to name only a few.

In one embodiment, the antibody or antigen-binding fragment is administered with a prefilled syringe. In another embodiment, the antibody is administered with a prefilled syringe containing a safety system. For example, the safety system prevents an accidental needlestick injury. In various embodiments, the antibody is administered with a prefilled syringe containing an ERIS^{™} safety system (West Pharmaceutical Services Inc.). See also U.S. patent numbers 5,215,534 and 9,248,242, incorporated herein by reference in their entireties.

In another embodiment, the antibody or antigen-binding fragment is administered with an auto-injector. In various embodiments, the antibody is administered with an auto-injector featuring the PUSHCLICK^{™} technology (SHL Group). In various embodiments, the auto-injector is a device comprising a syringe that allows for administration of a dose of the composition and/or antibody to a subject. See also U.S. patent numbers 9,427,531 and 9,566,395, incorporated herein by reference in their entireties.

In one embodiment, the antibody molecule or antigen-binding fragment inhibits, reduces or neutralizes or blocks fibrosis in a subject. The subject can be a mammal, e.g., a primate, preferably a higher primate, e.g., a human (e.g., a patient having, or at risk of having, a disorder described herein). In one embodiment, the subject is in need of inhibiting, reducing, neutralizing or blocking fibrosis or a fibrotic condition.

According to the invention, "subject" means a human subject or human patient.

According to the present invention "inhibiting a disease" means inhibiting or slowing the full development of a disease. In several examples, inhibiting a disease refers to lessening symptoms of a stress-mediated senescence/inflammation and/or fibrosis, such as the formation of scar tissue or an increase in range of motion or a decrease in pain. In some embodiments, inhibiting a disease refers to reducing or slowing progression of a stress-mediated senescence/inflammation and/or fibrosis. "Treatment" refers to a therapeutic intervention that ameliorates a sign or symptom of a disease or pathological condition related to the disease, such as the stress-mediated senescence/inflammation and/or fibrosis, and/or slowing progression of the disease or pathological condition related to the disease, such as stress-mediated senescence/inflammation and/or fibrosis.

According to certain embodiments of the present invention, "fibrosis" is the formation or development of excess fibrous connective tissue in an organ or tissue as a reparative or reactive process, as opposed to a formation of fibrous tissue as a normal constituent of an organ or tissue. In certain embodiments of the present invention fibrosis is the accumulation of fibrous connective tissue in and around inflamed or damaged tissue, which can lead to permanent scarring, organ malfunction and, ultimately, death. Fibrosis is the major histopathologic feature of a variety of clinical conditions and a pathological feature of most chronic inflammatory diseases. In the context of the present invention for vascular stress-mediated senescence is intended endothelial senescence and/or dysfunction, which lead to a vasoconstrictive, proinflammatory and prothrombotic environment.

An exemplary embodiment of vascular stress-mediated senescence comprises age-related conditions such as age-related cardiovascular diseases, including stroke, vascular dementia, macular degeneration, obstructive sleep apnea, atherosclerosis, myocardial infarction, pulmonary hypertension, hypertension, diabetes, renal failure, peripheral arterial disease, erectile dysfunction and diabetic foot.

In the context of the present invention for vascular inflammation is intended an inflammatory process that targets primarily the vessel district and includes endothelial inflammatory damage, infiltration of immune cells into the vessel wall. In an exemplary embodiment, vascular inflammation is selected from the group of conditions such as vasculitis, psoriasis, atherosclerosis, cardiovascular disease, peripheral arterial disease, myocardial infarction, vascular dementia, diabetes, renal failure.

In the context of the present invention for vascular fibrosis or fibrotic condition is intended an abnormal wound healing in blood vessels, leading to the excessive production of collagen-secreting fibroblasts, which can result in chronic diseases and organ failure. For example, vascular fibrosis may be found in skin lesions of psoriasis, vascular disease, vasculitis, heart failure, lung failure, atherosclerosis, cardiovascular disease, peripheral arterial disease, myocardial infarction, vascular dementia, diabetes, renal failure.

In the context of the present invention for acute stress cellular damage is intended an increase in cell death, upregulation of apoptotic markers, increased apoptosis upon treatment with stress factors (pro-inflammatory factors, oxidative factors, high glucose, serum inflammatory protein) and in prone-to-stress environment. For example, acute stress cellular damage may be found in all the aforementioned conditions.

In the context of the present invention for acute kidney injury is intended decrease in kidney function (glomerular filtration rate), resulting in the retention of urea and other nitrogenous waste products and in the dysregulation of extracellular volume and electrolytes. In an exemplary embodiment, acute kidney injury is selected from the group of conditions related to increased creatinine and reduction in urinary volume such as heart failure, liver failure, sepsis, dehydration, glomerulonephritis, vasculitis, contrast medium related kidney failure.

The antibodies and antigen-binding fragments disclosed herein can be used to treat stress-mediated senescence, inflammation, and fibrosis or fibrotic condition, and acute stress cellular damage, including cell apoptosis and death, occurring in several disease conditions. The antibodies and antigen-binding fragments disclosed herein may decrease stress-mediated senescence, apoptosis, inflammation, and fibrosis or fibrotic condition, and acute stress cellular damage that has already occurred, resolving existing fibrosis, and/or may decrease the rate or amount of additional fibrosis. In several examples, the antibodies and antigen-binding fragments are of use to decrease stress-mediated senescence, inflammation, and fibrosis or fibrotic condition, and acute stress cellular damage in pathogenic processes, such as in a subject. Thus, in several embodiments, the methods include administering to a subject a therapeutically effective amount of one or more of the antibodies disclosed herein, or antigen-binding fragment thereof in order to decrease stress-mediated senescence, inflammation, and fibrosis or fibrotic condition, and acute stress cellular damage. Any of the antibodies disclosed herein can be used to decrease stress-mediated senescence, inflammation, and fibrosis or fibrotic condition, and acute stress cellular damage. In some embodiments, the antibodies and antigen-binding fragments thereof can be administered as a unit dose.

Suitable subjects include those with stress-mediated senescence, inflammation, and fibrosis or fibrotic condition, and acute stress cellular damage, preferably wherein said vascular stress-mediated senescence, inflammation, and fibrosis or fibrotic condition is selected from psoriasis, heart failure or vasculopathy; and wherein said acute stress cellular damage is acute kidney injury. Stress-mediated senescence, inflammation, and fibrosis or fibrotic condition, and acute stress cellular damage of any tissue can be treated using the methods disclosed herein.

In further examples, the methods are used to treat stress-mediated senescence, inflammation, and fibrosis or fibrotic condition, and acute stress cellular damage. The methods can include selecting a subject in need of treatment, such as a subject with a stress-mediated senescence, inflammation, and fibrosis or fibrotic condition, and acute stress cellular damage, or any of the disorders listed above, in an amount sufficient to reduce the stress-mediated senescence, inflammation, and fibrosis or fibrotic condition, and acute stress cellular damage. Amounts effective for this use will depend upon the severity of the disease, the general state of the patient's health, and the robustness of the patient's immune system. In one example, a therapeutically effective amount of the compound is that which provides either subjective relief of a symptom(s) or an objectively identifiable improvement as noted by the clinician or other qualified observer. A method is provided herein for decreasing stress-mediated senescence, inflammation, and fibrosis or fibrotic condition, and acute stress cellular damage. The method includes administering a therapeutically effective amount of an anti- IGFBP7 antibody, such as those disclosed herein, or antigen-binding fragment thereof, thereby decreasing stress-mediated senescence, inflammation, and fibrosis or fibrotic condition, and acute stress cellular damage.

An antibody herein disclosed, or antigen-binding fragment thereof, can be administered by any means known to one of skill in the art either locally or systemically, such as by intradermal, intrathecal, intramuscular, subcutaneous, intraperitoneal or intravenous injection, but even oral, nasal, transdermal or anal administration is contemplated. In one embodiment, administration is by subcutaneous, intradermal, or intramuscular injection. In another embodiment, administration is by intraperitoneal or intrathecal administration. To extend the time during which the antibody is available to stimulate a response, the antibody or antigen-binding fragment thereof can be provided as an implant, an oily injection, or as a particulate system. The particulate system can be a microparticle, a microcapsule, a microsphere, a nanocapsule, or similar particle, (see, e.g., Banga, supra).

For treatment of the skin, a therapeutically effective amount of the antibody or antigen-binding fragment thereof can be locally administered to the affected area of the skin, such as in the form of an ointment (RGA Jones and A Marino, "Targeted localized use of therapeutic antibodies: a review of non-systemic, topical and oral applications, Crit. Rev. Biotechnol. 36(3):506-520 (2016). In one embodiment, the ointment is an entirely homogenous semi-solid external agent with a firmness appropriate for easy application to the skin. Such an ointment can include fats, fatty oils, lanoline, Vaseline, paraffin, wax, hard ointments, resins, plastics, glycols, higher alcohols, glycerol, water or emulsifier and a suspending agent. Using these ingredients as a base, a decoy compound can be evenly mixed. Depending on the base, the mixture can be in the form of an oleaginous ointment, an emulsified ointment, or a water-soluble ointment oleaginous ointments use bases such as plant and animal oils and fats, wax, Vaseline and liquid paraffin. Emulsified ointments are comprised of an oleaginous substance and water, emulsified with an emulsifier. They can take either an oil-in-water form (O/W) or a water-in-oil-form (W/O). The oil-in-water form (O/W) can be a hydrophilic ointment. The water-in-oil form (W/O) initially lacks an aqueous phase and can include hydrophilic Vaseline and purified lanoline, or it can contain a water-absorption ointment (including an aqueous phase) and hydrated lanoline. A water-soluble ointment can contain a completely water-soluble Macrogol base as its main ingredient.

Pharmaceutically acceptable carriers include a petroleum jelly, such as VASELlNE^{®}, wherein the petroleum jelly contains 5% stearyl alcohol, or petroleum jelly alone, or petroleum jelly containing liquid paraffin. Such carriers enable pharmaceutical compositions to be prescribed in forms appropriate for consumption, such as tablets, pills, sugar-coated agents, capsules, liquid preparations, gels, ointments, syrups, slurries, and suspensions. When locally administered into cells in an affected area or a tissue of interest, the at least one C-terminal endostatin polypeptide, or polynucleotide encoding the peptide can be administered in a composition that contains a synthetic or natural hydrophilic polymer as the carrier. Examples of such polymers include hydroxypropyl cellulose and polyethylene glycol. One or more antibody can be mixed with a hydrophilic polymer in an appropriate solvent. The solvent is then removed by methods such as air-drying, and the remainder is then shaped into a desired form (for example, a sheet) and applied to the target site. Formulations containing such hydrophilic polymers keep well as they have a low water-content. At the time of use, they absorb water, becoming gels that also store well. In the case of sheets, the firmness can be adjusted by mixing a polyhydric alcohol with a hydrophilic polymer similar to those above, such as cellulose, starch and its derivatives, or synthetic polymeric compounds. Hydrophilic sheets thus formed can be used. A therapeutically effective amount of one or more antibodies can also be incorporated into bandages and dressings.

For administration by inhalation, the antibody or antigen-binding fragment thereof can be conveniently delivered in the form of an aerosol spray presentation from pressurized packs or a nebulizer, with the use of a suitable propellant, such as dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges for use in an inhaler or insufflator can be formulated containing a powder mix of the compound and a suitable powder base such as lactose or starch. In some embodiments, the antibody or antigen-binding fragment thereof can be administered by inhalation. For example, it can be administered in an aerosolized form, such as using a nebulizer or a metered dose inhaler. Technologies of use include micropump nebulizers (such as the AEROGEN GO^{®} system), jet nebulizers designed to produce large fine particle fractions (such as the PARI LC STAR^{®}), jet nebulizers developing less shear during atomization (such as the HUDSON MICROMIST^{®}), and ultrasonic nebulizers (such as the DeVilbiss ULTRA-NEB^{®}). The antibody or antigen-binding fragment thereof can be dissolved in a carrier, such as saline, and atomized using the devices above. The associated aerosols can be collected using a NEXT GENERATION IMPACTOR^{®} (NGI) (MSP Corp., Shoreview, Minn.), which uses a series of aerodynamic stages to separate and collect the aerosol into separate fractions based on droplet size.

Aerosol particle size is often expressed in terms of mass median aerodynamic diameter (MMAD), a parameter that is based on particle size, shape, and density. For a spherical particle, MMAD is equal to MMD (p<1/2>), in which MMD is mass median diameter and r is the bulk density. For a non-spherical particle, MMAD is equal to MMD (p/x)<1/2>, in which X is the shape factor. Thus, particles with larger than unit density will have actual diameters smaller than their MMAD.

The site of particle deposition within the respiratory tract is demarcated based on particle size. In one example, particles of about 1 to about 500 microns are utilized, such as particles of about 25 to about 250 microns, or about 10 to about 25 microns are utilized. In other embodiments, particles of about 1 to 50 microns are utilized. For use in a metered dose inhaler, for administration to lungs particles of less than about 10 microns, such as particles of about 2 to about 8 microns, such as about 1 to about 5 microns, such as particles of 2 to 3 microns, can be utilized.

A therapeutically effect amount of an antibody or antigen-binding fragment thereof can be administered in the pharmaceutically acceptable carrier. Pharmacologically acceptable carriers (e.g., physiologically or pharmaceutically acceptable carriers) are well known in the art, and include, but are not limited to buffered solutions as a physiological pH (e.g. from a pH of about 7.0 to about 8.0, or at a pH of about 7.4). One specific, non-limiting example of a physiologically compatible buffered solution is phosphate buffered saline. Other pharmacologically acceptable carriers include penetrants, which are particularly suitable for pharmaceutical formulations that are intended to be topically applied (for example in the application of surgical wounds to promote healing).

The pharmacological compositions disclosed herein facilitate the use of at least one antibody or antigen-binding fragment thereof, either in vivo or ex vivo, to decrease stress-mediated senescence, inflammation, and fibrosis or fibrotic condition, and acute stress cellular damage. Such a composition can be suitable for delivery of the active ingredient to any suitable subject, and can be manufactured in a manner that is itself known, e.g., by means of conventional mixing, dissolving, granulating, emulsifying, encapsulating, entrapping or lyophilizing processes. Pharmacological compositions can be formulated in a conventional manner using one or more pharmacologically (e.g., physiologically or pharmaceutically) acceptable carriers, as well as optional auxiliaries that facilitate processing of the active compounds into preparations which can be used pharmaceutically. Proper formulation is dependent upon the route of administration chosen. Thus, for injection, the active ingredient can be formulated in aqueous solutions. For transmucosal administration, penetrants appropriate to the barrier to be permeated are used in the formulation. Such penetrants are generally known in the art.

For oral administration, the active ingredient can be combined with carriers suitable for incorporation into tablets, pills, capsules, liquids, gels, syrups, slurries, suspensions and the like. The active ingredient can be formulated for parenteral administration by injection, such as by bolus injection or continuous infusion. Such compositions can take such forms as suspensions, solutions or emulsions in oily or aqueous vehicles, and can contain formulatory agents such as suspending, stabilizing and/or dispersing agents. Other pharmacological excipients are known in the art.

Other delivery systems can include time-release, delayed release or sustained release delivery systems. Such systems can avoid repeated administrations of the compositions of the invention described above, increasing convenience to the subject and the physician. Many types of release delivery systems are available and known to those of ordinary skill in the art. They include polymer-based systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems, such as lipids including sterols such as cholesterol, cholesterol esters and fatty acids or neutral fats such as mono- di- and triglycerides; hydrogel release systems; silastic systems; peptide based systems; wax coatings; compressed tablets using conventional binders and excipients; partially fused implants; and the like. Specific examples include, but are not limited to: (a) erosional systems in which the at least one C-terminal endostatin polypeptide, or polynucleotide encoding the peptide is contained in a form within a matrix such as those described in U.S. Pat. Nos. 4,452,775; 4,667,014; 4,748,034; 5,239,660; and 6,218,371 and (b) diffusional systems in which an active component permeates at a controlled rate from a polymer such as described in U.S. Pat. Nos. 3,832,253 and 3,854,480. In addition, pump-based hardware delivery systems can be used, some of which are adapted for implantation.

The therapeutically effective amount of the antibody or antigen-binding fragment thereof will be dependent on the antibody that is utilized, the subject being treated, the severity and type of the affliction, and the manner of administration. For example, a therapeutically effective amount of an antibody can vary from about 0.01 µg per kilogram (kg) body weight to about 1 g per kg body weight, such as about 1 µg to about 5 mg per kg body weight, or about 5 µg to about 1 mg per kg body weight. The exact dose is readily determined by one of skill in the art based on the potency of the specific compound the age, weight, sex and physiological condition of the subject.

Single or multiple administrations of the compositions are administered depending on the dosage and frequency as required and tolerated by the subject. In one embodiment, the dosage is administered once as a bolus, but in another embodiment can be applied periodically until a therapeutic result is achieved. Generally, the dose is sufficient to treat or ameliorate symptoms or signs of disease without producing unacceptable toxicity to the subject. Systemic or local administration can be utilized.

In a further method, an additional agent is administered. In one example, this administration is sequential. In other examples, the additional agent is administered simultaneously with the antibody.

The disclosure is illustrated by the following non-limiting Examples.

### EXAMPLES

### Example 1

### MONOCLONAL ANTIBODIES DEVELOPMENT

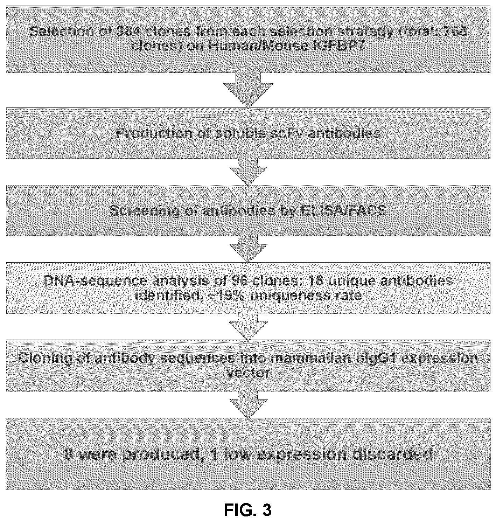

Monoclonal anti-IGFBP7 antibodies were discovered from naive human phage-display libraries using a recombinant full length human IGFBP7 as antigen for the screening. Briefly human IGFBP7 (SinoBiological, 13100-H07H5) was biotinylated and immobilized via streptavidin onto 96-well ELISA plates at 4 °C. After washing and blocking of the wells with BSA, the antibody-phage libraries were added. These were cleared previously from antibody-phage that bind to Streptavidin. Those phages that carried an antigen-specific antibody were captured on the plate surface. After removal of unbound/weakly bound phage with PBS-T, antigen-specific phage was eluted and amplified in E. coli. This amplified library subset was again selected for target binding under more stringent conditions. In total, three selection rounds were performed to enrich antigen specific antibody-phage. At the end of the discovery process, each selection output was screened for antigen-specific antibodies. For this purpose, 384 clones were chosen from each selection output and used for production of monoclonal scFv antibodies **(****Figure 3**). These were then tested for specific antigen binding by ELISA. All the unique antibodies were cloned into a mammalian scFv-Fc expression vector. As a result, the scFv was genetically fused to a Fc Fragment of human IgG1.

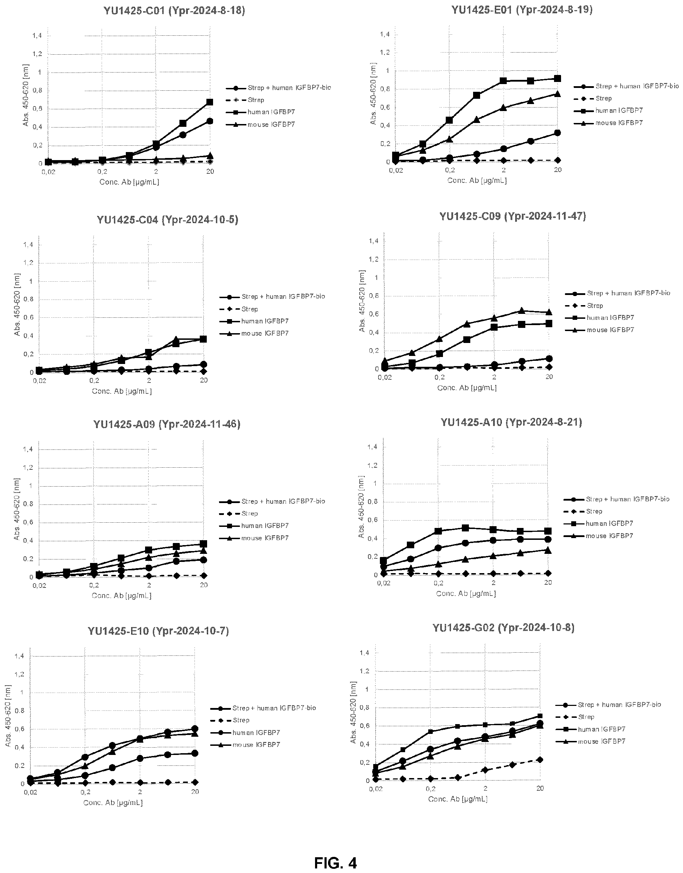

Finally, 8 scFv-Fc antibodies were produced by transient transfection of HEK293 cells **(****Figure 4****).** Then, the antibodies were purified by affinity chromatography (Protein A) and re-buffered in PBS (no additives). The protein concentration was determined by UV/VIS spectrometry and purity was checked by Coomassie staining **(Table 8).**

### Example 2

### RESULTS

### Recombinant human IGFBP7 mediates apoptosis and cell death in kidney tubular cells

Human tubular kidney cells (HK2) were cultured in the presence or absence of recombinant human IGFBP7 (rhlGFBP7) in a dose-dependent manner (20, 150, 300 ng/ml) for 48 hours and analyzed for cell death and Caspase3/7 (Casp3/7) apoptosis. rh!GFBP7 increased cell death at concentrations 150 and 300 ng/ml and confirmed its negative effect on tubular cells *in vitro*

### (Figure 1).

### Recombinant human IGFBP7 mediates apoptosis and cell death in cardiomyocytes

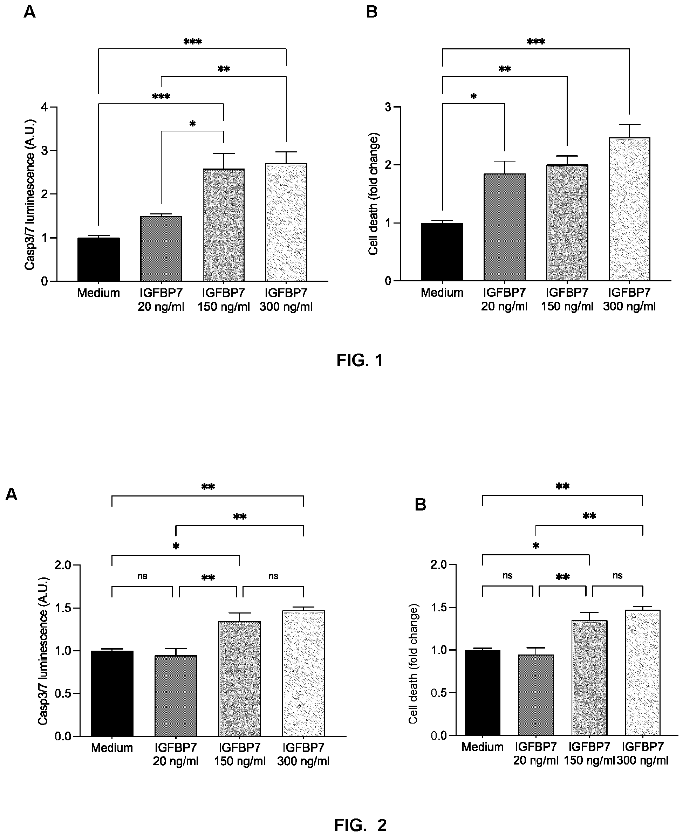

Human cardiomyocytes (AC16) were differentiated for 7 days as per manufacturer's protocol and cultured in the presence or absence of rhlGFBP7 in a dose-dependent manner (20, 150, 300 ng/ml) for 48 hours. The analysis of cell death and Caspase3/7 assay revealed an increased apoptosis and cell death in presence of rhlGFBP7 mainly at the higher concentrations (150 and 300 ng/ml) and confirmed its negative effect on cardiac differentiated cells *in vitro* **(****Figure 2****).**

### Monoclonal antibodies (mAbs) directed against IGFBP7 have been generated by phage display

We have screened, generated and produced monoclonal antibodies by phage display screening and generation procedure. 384 clones were selected to bind to Human/mouse IGFBP7, produced as scFv antibodies and screened for binding by ELISA. DNA-sequence analysis of the top 96 clones identified 18 unique antibodies which were cloned into mammalian human IgG1 expression vector and eight antibodies were produced, one with very low expression (discarded). A schematic strategy is presented in Figure 3.

### Anti-IGFBP7 mAbs generated by phage display bind human and murine immobilized IGFBP7 antigens

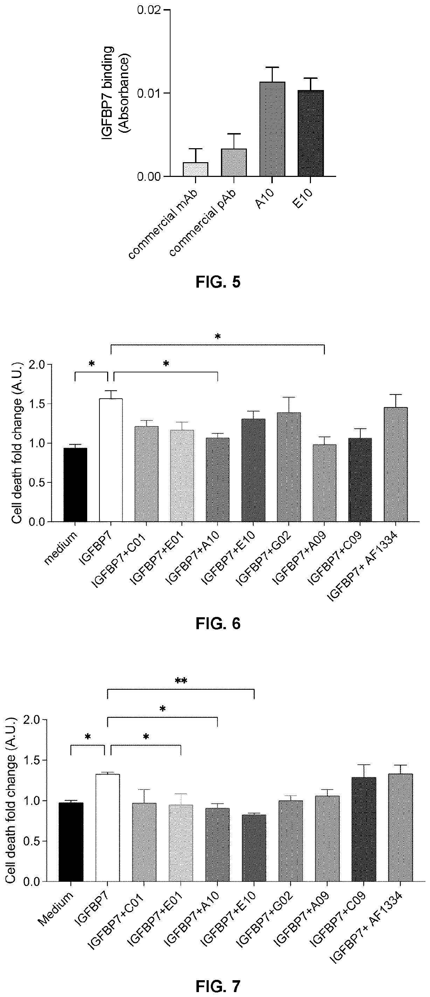

The seven anti-IGFBP7 mAbs produced by phage display were next screened for their ability to bind immobilized human IGFBP7, immobilized murine IGFBP7, streptavidin conjugated soluble human IGFBP7. IGFBP7 and the available antibodies were all used in a 1:1 ratio. All the seven antibodies tested were able to bind human IGFBP7 and to murine IGFBP7 directly immobilized by ELISA, although the binding to murine IGFBP7 was lower. The mAbs E10, E01 and A10 showed higher binding to IGFBP7 **(****Figure 4****,** blacklines). In vitro ELISA binding assay was also performed to compare binding between the newly generated anti-IGFBP7 mAbs and the commercially available monoclonal (ab171085 AbCam) and polyclonal (AF1334 R&D Systems) Abs. The mAbs A10 and E10 showed higher binding also compared to commercially available antibodies **(****Figure 5****).**

***Anti-IGFBP7 mAbs EC50 analysis***

Half maximal effective concentration (EC50) was also calculated for the seven mAbs produced and A10 showed the best EC50 for human IGFBP7, both for directly immobilized protein as well as for streptavidin conjugated soluble human protein **(Table 9).** Concentration tested 20 ug/ml.

### Newly generated monoclonal anti-IGFBP7 antibodies prevent cell death in kidney human tubular cells

The seven newly generated monoclonal antibodies were tested in a cell death assay in which kidney tubular cells HK2 were challenged with rhlGFBP7 (150 ng/ml). Tubular cells were cultured for 48 hours upon IGFBP7 exposure and treated with the newly generated anti-IGFBP7 mAbs at a ratio of 1:1. Appropriate control antibodies were also included with the same ratio (polyclonal goat IgG, R&D, AF1334). We observed that four out of seven (C01, A10, A09, C09) rescued IGFBP7 mediated tubular cell death and in particular A09 and A10 appeared more powerful as compared to the polyclonal anti-IGFBP7 control **(****Figure 6****,** p<0.05).

### Newly generated monoclonal anti-IGFBP7 antibodies prevent cell death in human endothelial cells

Seven newly generated monoclonal antibodies were tested in a cell death assay in which human endothelial cells (HUVEC) were challenged with rhlGFBP7 (150 ng/ml). Cells were cultured for 48 hours upon IGFBP7 exposure and treated with the newly generated anti-IGFBP7 mAbs at a ratio of 1:1. Appropriate control antibodies were also included with the same ratio (polyclonal goat IgG, R&D, AF1334). We observed that three out of seven (E01, A10, E10) rescued IGFBP7 mediated cell death **(****Figure 7****,** p<0.05).

### Example 3

### METHODS

### Cells

Human tubular cells (HK2) were purchased from ATCC (CRL-2190^{™}) and cultured as per manufacturer's instructions (Invitrogen - GIBCO) by using the kit Keratinocyte Serum Free Medium (Invitrogen - GIBCO, 17005042) supplemented with 0.05 mg/ml BPE and 5 ng/ml EGF. HUVECs (Lonza C2519A) were cultured in endothelial growth medium provided by the vendor (Lonza CC-3162). AC16 cardiomyocytes were purchased from Sigma-Aldrich (SCC109) and cultured as per manufacturer's instructions in mitogen-depleted medium. At 7 days the AC16 cells stopped proliferating and differentiated in cardiac muscle cells.

### Recombinant proteins and interventional studies

Recombinant human IGFBP7 (SinoBiological, 13100-H07H5), 50 ng/ml, 150 ng/ml and 300 ng/ml was added to cell cultures. Newly generated monoclonal and the commercial polyclonal anti-IGFBP7 antibody (R&D Systems) were added at 20 ug/ml at 1:1 molecular ratio as compared to rhlGFBP7.

### Binding assay

The following reagents were used to screen the anti-IGFBP7 antibodies: Recombinant Human IGFBP-7 (0,1, 1 and 10 ug/ml SinoBiological), newly generated human anti-IGFBP7 mAbs A10 and E10 (10 ug/ml), goat polyclonal anti-IGFBP7 (AF1334, R&D Systems), rabbit monoclonal anti-IGFBP7 (ab171085, AbCam), mouse 6X His tag HRP (Genetex), bovine serum albumin (BSA), Tween 20 (TW), ELISA colorimetric TMB reagent (HRP substrate, Item H Sigma, RABTMB3), ELISA STOP solution (Item I, Sigma, RABSTOP3), blocking reagent solution(3%BSA in PBS) and a diluent solution (0,5%BSA, 0,05%Tw in PBS). CVC microplate was coated with 10 µg/ml of the above listed antibodies dissolved in PBS or PBS alone (no coating). Plate was incubated 90 minutes at 37°C and washed with PBS (300 µl/well) and incubated with the blocking reagent (200 µl/well) for 2 hours at room temperature. Samples were then diluted in the diluent solution (50 µl/well) and rhlGFBP7 was added to the plate at concentration of 0, 1, 1 and 10 ug/ml. ELISA plate was then read after adding visualization solution at ELISA reader.

### Cell death assay

Cell lysates were collected after 48 hours, and cell death/apoptosis was assessed by using ELISA (Roche Diagnostics GmbH, 11544675001, Mannheim, Germany). Quantification of cell death was normalized to untreated cells.

### Caspase 3/7 luminescence assay

Cells were cultured with recombinant hlGFBP7 with or without anti-IGFBP7 mAbs as described above, collected and transferred to an opaque-walled 96-well plate. Caspase 3/7 activity was quantified using the Caspase-Glo 3/7 assay (Promega #G8091). The single caspase-Glo reagent allows cell lysis and provides a luminogenic caspase 3/7 substrate, which upon cleavage, generates a luminescent signal. Luminescence is proportional to the amount of substrate cleaved, thus caspase 3/7 activity. According to manufacturer's instruction, 100ul of single reagent were added to 100ul of cell suspensions and after 30 minutes luminescence was quantified using the GloMax instrument.

### Statistical analysis

Data are presented as mean and standard error of the mean (SEM). The statistical significance of differences was tested with two-tailed t-test test. Significance between the two groups was determined by two-tailed unpaired Student's t test. For multiple comparisons, the ANOVA test with Sidak correction was employed. All data were analyzed by GraphPad Prism V9 (GraphPad Software, La Jolla, CA). All statistical tests were performed at the 5% significance level.

**TABLES**

| **Table 1: VH CDR sequences of exemplified antibodies** | | | |
|---|---|---|---|
| **Antibody** | **VH CDR1** | **VH CDR2** | **VH CDR3** |
| YU1425-A10 | DYAMH (SEQ ID NO. 1) | GISWNSGSIGYADSVKG (SEQ ID NO. 2) | GLVAATTPPYFDY (SEQ ID NO. 8) |
| YU1425-A09 | DYAMH (SEQ ID NO. 1) | GISWNSGSIGYADSVKG (SEQ ID NO. 2) | DISGSQLVLLDY (SEQ ID NO. 4) |
| YU1425-E10 | DYAMH (SEQ ID NO. 1) | GISWNSGSIGYADSVKG (SEQ ID NO. 2) | ANYYDSSALDY (SEQ ID NO. 3) |
| YU1425-C09 | SYAMH (SEQ ID NO. 14) | AISSNGGSTYYADSVKG (SEQ ID NO. 15) | GEQWLAYNWFDP (SEQ ID NO. 16) |
| YU1425-C01 | SYSMS (SEQ ID NO. 29) | AISGSGGTYYADSVKG (SEQ ID NO. 30) | SLRAFSY (SEQ ID NO. 31) |
| YU1425-E01 | SYWIG (SEQ ID NO. 32) | IIYPGDSDTRYSPSFQG (SEQ ID NO. 33) | LGVSGSYRRSGMDV (SEQ ID NO. 34) |
| YU1425-G02 | SYAIS (SEQ ID NO. 9) | RIIPIFGIANYAQKFQG (SEQ ID NO. 13) | RFQH (SEQ. ID No. 128) |
| YU1425-G11 | DYAMH (SEQ ID NO. 1) | GISWNSGSIGYADSVKG (SEQ ID NO. 2) | DIWATVTPAAFDY (SEQ ID NO. 5) |
| YU1425-C04 | DYAMH (SEQ ID NO. 1) | GISWNSGSIGYADSVKG (SEQ ID NO. 2) | DNSGSGRYFDY (SEQ ID NO. 6) |
| YU1425-A01 | DYAMH (SEQ ID NO. 1) | GISWNSGSIGYADSVKG (SEQ ID NO. 2) | DVTAMDTVGFQH (SEQ ID NO. 7) |
| YU1425-G09 | SYAIS (SEQ ID NO. 9) | GIIPIFGTANYAQKFQG (SEQ ID NO. 10) | GTYYYDSSGTPDY (SEQ ID NO. 11) |
| YU1425-H11 | SYAIS (SEQ ID NO. 9) | GIIPIFGTANYAQKFQG (SEQ ID NO. 10) | PSVLEWTYYYYYGM DV (SEQ ID NO. 12) |
| YU1425-F05 | SYAMH (SEQ ID NO. 14) | VISYDGSNKYYADSVKG (SEQ ID NO. 17) | DSGPTTVTTFDY (SEQ ID NO. 18) |
| YU1425-A12 | SYAMH (SEQ ID NO. 14) | VISYDGSNKYYADSVKG (SEQ ID NO. 17) | SVGYLNWFDP (SEQ ID NO. 19) |
| YU1425-B08 | SYAMS (SEQ ID NO. 20) | AISGSGGSTYYADSVKG (SEQ ID NO. 21) | DHPPSVYSSSWLRFDY (SEQ ID NO. 22) |
| YU1425-A04 | SYAMS (SEQ ID NO. 20) | AISGSGGSTYYADSVKG (SEQ ID NO. 21) | DLFLDLYDSSGYYLVPQIH (SEQ ID NO. 23) |
| YU1425-B05 | SYGMH (SEQ ID NO. 24) | VISYDGSNKYYADSVKG (SEQ ID NO. 17) | HGDYGGYQYFQH (SEQ ID NO. 25) |
| YU1425-C08 | SYSMN (SEQ ID NO. 26) | SISSSSSYIYYADSVKG (SEQ ID NO. 27) | DRDTYYDYGDNFDY (SEQ ID NO. 28) |

CDRs are defined according to Kabat.

| **Table 2: VL CDR sequences of exemplified antibodies** | | | |
|---|---|---|---|
| **Antibody** | **VL_CDR1** | **VL_CDR2** | **VL_CDR3** |
| YU1425-A10 | TGTSSDVGGYNYVS (SEQ ID NO. 35) | DVSNRPS (SEQ ID NO. 36) | SSYTSSSTLV (SEQ ID NO. 46) |
| YU1425-A09 | TGSSSNIGAGYDVH (SEQ ID NO. 38) | GNSNRPS (SEQ ID NO. 39) | QSYDSSLSVV (SEQ ID NO. 40) |
| YU1425-E10 | TGTSSDVGGYNYVS (SEQ ID NO. 35) | DVSNRPS (SEQ ID NO. 36) | SSYTSSSTWV (SEQ ID NO. 37) |
| YU1425-C09 | TGSSSNIGAGYDVH (SEQ ID NO. 38) | GNSNRPS (SEQ ID NO. 39) | QSYDSSLSGWGV (SEQ ID NO. 56) |
| YU1425-C01 | RSSQSLLHSNGYNYLD (SEQ ID NO. 41) | LGSNRAS (SEQ ID NO. 42) | MQSLRTPLT (SEQ ID NO. 69) |
| YU1425-E01 | RASQSVSSSYLA (SEQ ID NO. 70) | GASSRAT (SEQ ID NO. 71) | QQYGSSPLT (SEQ ID NO. 72) |
| YU1425-G02 | SGSSSNIGSNTVN (SEQ ID NO. 53) | SNNQRPS (SEQ ID NO. 54) | AAWDDSLNGYV (SEQ ID NO. 55) |
| YU1425-G11 | RSSQSLLHSNGYNYLD (SEQ ID NO. 41) | LGSNRAS (SEQ ID NO. 42) | MQALQTPL (SEQ ID NO. 43) |
| YU1425-C04 | TGTSSDVGGYNYVS (SEQ ID NO. 35) | DVSNRPS (SEQ ID NO. 36) | SSYTSSSTLVV (SEQ ID NO. 44) |
| YU1425-A01 | RSSQSLLHSNGYNYLD (SEQ ID NO. 41) | LGSNRAS (SEQ ID NO. 42) | MQALQTPYT (SEQ ID NO. 45) |
| YU1425-G09 | SGSSSNIGNNYVS (SEQ ID NO. 47) | DNNKRPS (SEQ ID NO. 48) | GTWDSSLSAVV (SEQ ID NO. 49) |
| YU1425-H11 | QASQDISNYLN (SEQ ID NO. 50) | DASNLET (SEQ ID NO. 51) | QQYDNLPLT (SEQ ID NO. 52) |
| YU1425-F05 | SGSSSNIGSNYVY (SEQ ID NO. 57) | RNSQRPS (SEQ ID NO. 58) | AAWDDSLSVPYV (SEQ ID NO. 59) |
| YU1425-A12 | RASQSISSWLA (SEQ ID NO. 60) | KASSLES (SEQ ID NO. 61) | QQYNSYLIT (SEQ ID NO. 62) |
| YU1425-B08 | TGSSSNIGAGYDVH (SEQ ID NO. 38) | GNSNRPS (SEQ ID NO. 39) | QSYDSSLSGFVV (SEQ ID NO. 63) |
| YU1425-A04 | TGTSSDVGGYNYVS (SEQ ID NO. 35) | DVSNRPS (SEQ ID NO. 36) | SSYTSSSTVV (SEQ ID NO. 64) |
| YU1425-B05 | TGSSSNIGAGYDVH (SEQ ID NO. 38) | GNSNRPS (SEQ ID NO. 39) | QSYDSSLSGSWV (SEQ ID NO.65) |
| YU1425-C08 | SGDALPKQYAY (SEQ ID NO. 66) | KDSERPS (SEQ ID NO. 67) | QSADSSGTYYV (SEQ ID NO. 68) |

CDRs are defined according to Kabat.

| **Table 3: VH amino acid sequences of exemplified antibodies** | |
|---|---|
| **Antibody** | **AA of VH** |
| YU1425-A10 | EVQLVQSGGGLVQPGRSLRLSCAASGFTFDDYAMHWVRQAPGKGLEWVSG ISWNSGSIGYADSVKGRFTISRDNAKNSLYLQMNSLRAEDTALYYCAKGLVAA TTPPYFDYWGQGTLVTVSS (SEQ ID NO. 78) |
| YU1425-A09 | QVQLVQSGGGLVQPGRSLRLSCAASGFTFDDYAMHWVRQAPGKGLEWVS GISWNSGSIGYADSVKGRFTISRDNAKNSLYLQMNSLRAEDTALYYCAKDISG SQLVLLDYWGQGTTVTVSS (SEQ ID NO. 74) |
| YU1425-E10 | EVQLLESGGGLVQPGRSLRLSCAATGFTFDDYAMHWVRQAPGKGLEWVSGI SWNSGSIGYADSVKGRFTISRDNAKNSLYLQMNSLRAEDTALYYCAKANYYD SSALDYWGQGTLVTVSS (SEQ ID NO. 73) |
| YU1425-C09 | EVQLLESGGGLVQPGGSLRLSCSASGFTFSSYAMHWVRQAPGKGLEYVSAI SSNGGSTYYADSVKGRFTISRDNSKNTLYLQMSSLRAEDTAVYYCVKGEQW LAYNWFDPWGQGTTVTVSS (SEQ ID NO. 82) |
| YU1425-C01 | QVQLVQSGGGLVQPGGSLRLSCAASGFTFSSYSMSWVRQAPGKGLEWVSA ISGSGGTYYADSVKGRFTISRDSSKNTLYLQMNSLRAEDTAVYYCARSLRAFS YWGQGTLVTVSS (SEQ ID NO. 89) |
| YU1425-E01 | QVQLVQSGAEVKKPGESLKISCKGSGYSFTSYWIGWVRQMPGKGLEWMGII YPGDSDTRYSPSFQGQVTISADKSISTAYLQWSSLKASDTAMYYCARLGVSG SYRRSGMDVWGQGTLVTVSS (SEQ ID NO. 90) |
| YU1425-G02 | QVQLVQSGAEVKKPGSSVKVSCKASGGTFSSYAISWVRQAPGQGLEWMGRI IPIFGIANYAQKFQGRVTITADKSTSTAYMELSSLRSEDTAVYYCARRFQHWG QGTLVTVSS (SEQ ID NO. 81) |
| YU1425-G11 | QVQLVQSGGGLVQPGRSLRLSCAASGFTFDDYAMHWVRQAPGKGLEWVS GISWNSGSIGYADSVKGRFTISRDNAKNSLYLQMNSLRAEDTALYYCAKDIWA TVTPAAFDYWGQGTLVTVSS (SEQ ID NO. 75) |
| YU1425-C04 | QVQLVQSGGGLVQPGRSLRLSCAASGFTFDDYAMHWVRQAPGKGLEWVS GISWNSGSIGYADSVKGRFTISRDNAKNSLYLQMNSLRAEDTALYYCAKDNS GSGRYFDYWGQGTLVTVSS (SEQ ID NO. 76) |
| YU1425-A01 | QVQLVESGGGLVQPGRSLRLSCAASGFTFDDYAMHWVRQAPGKGLEWVSG ISWNSGSIGYADSVKGRFTISRDNAKNSLYLQMNSLRAEDTALYYCAKDVTA MDTVGFQHWGQGTLVTVSS (SEQ ID NO. 77) |
| YU1425-G09 | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCARGTYYYD SSGTPDYWGQGTTVTVSS (SEQ ID NO. 79) |
| YU1425-H11 | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCARPSVLEW TYYYYYGMDVWGQGTLVTVSS (SEQ ID NO. 80) |
| YU1425-F05 | QVQLVQSGGGVVQPGRSLRLSCAASGFTFSSYAMHWVRQAPGKGLEWVAV ISYDGSNKYYADSVKGRFTISRDNSKNTLYLQMNSLRAEDTAVYYCARDSGP TTVTTFDYWGQGTLVTVSS (SEQ ID NO. 83) |
| YU1425-A12 | QVQLVQSGGGVVQPGRSLRLSCAASGFTFSSYAMHWVRQAPGKGLEWVAV ISYDGSNKYYADSVKGRFTISRDNSKNTLYLQMNSLRAEDTAVYYCARSVGY LNWFDPWGQGTLVTVSS (SEQ ID NO. 84) |
| YU1425-B08 | QVQLVESGGGLVQPGGSLRLSCAASGFTFSSYAMSWVRQAPGKGLEWVSAI SGSGGSTYYADSVKGRFTISRDNSKNTLYLQMNSLRAEDTAVYYCAKDHPPS VYSSSWLRFDYWGQGTTVTVSS (SEQ ID NO. 85) |
| YU1425-A04 | EVQLVESGGGLVKPGGSLRLSCAASGFTFSSYAMSWVRQAPGKGLEWVSAI SGSGGSTYYADSVKGRFTISRDNSKNTLYLQMNSLRAEDTAVYYCAKDLFLD LYDSSGYYLVPQIHWGQGTLVTVSS (SEQ ID NO. 86) |
| YU1425-B05 | QVQLQESGGGVVQPGRSLRLSCAASGFTFSSYGMHWVRQAPGKGLEWVA VISYDGSNKYYADSVKGRFTISRDNSKNTLYLQMNSLRAEDTAVYYCATHGD YGGYQYFQHWGQGTTVTVSS (SEQ ID NO. 87) |
| YU1425-C08 | QVQLVQSGGGLVKPGGSLRLSCAASGFTFSSYSMNWVRQAPGKGLEWVSS ISSSSSYIYYADSVKGRFTISRDNAKNSLYLQMNSLRAEDTAVYYCARDRDTY YDYGDNFDYWGQGTLVTVSS (SEQ ID NO. 88) |

| **Table 4: VL amino acid sequences of exemplified antibodies** | |
|---|---|
| **Antibody** | **AA of VL** |
| YU1425-A10 | QSALTQPASVSGSPGQSITISCTGTSSDVGGYNYVSWYQQHPGKAPKLMIY DVSNRPSGVSNRFSGSKSGNTASLTISGLQAEDEADYYCSSYTSSSTLVFG GGTKVTVL (SEQ ID NO. 96) |
| YU1425-A09 | QSVLTQPPSVSGAPGQRVTISCTGSSSNIGAGYDVHWYQQLPGTAPKLLIYG NSNRPSGVPDRFSGSKSGTSASLAIAGLQAEDEADYYCQSYDSSLSVVFGG GTKVTVL (SEQ ID NO. 92) |
| YU1425-E10 | QSALTQPASVSGSPGQSITISCTGTSSDVGGYNYVSWYQQHPGKAPKLMIY DVSNRPSGVSNRFSGSKSGNTASLTISGLQAEDEADYYCSSYTSSSTWVFG GGTKLTVL (SEQ ID NO. 91) |
| YU1425-C09 | QSVLTQPPSVSGAPGQRVTISCTGSSSNIGAGYDVHWYQQLPGTAPKLLIYG NSNRPSGVPDRFSGSKSGTSASLAITGLQAEDEADYYCQSYDSSLSGWGVF GGGTKLTVL (SEQ ID NO. 100) |
| YU1425-C01 | DVVMTQSPLSLPVTPGEPASISCRSSQSLLHSNGYNYLDWYLQKPGQSPQL LIYLGSNRASGVPDRFSGSGSGTDFTLNISRVEAEDVGVYYCMQSLRTPLTF GGGTKLEIK (SEQ ID NO. 107) |
| YU1425-E01 | EIVLTQSPGTLSLSPGERATLSCRASQSVSSSYLAWYQQKPGQAPRLLIYGA SSRATGIPDRFSGSGSGTDFTLTISRLEPEDFAVYYCQQYGSSPLTFGGGTK LEIK (SEQ ID NO. 108) |
| YU1425-G02 | SYVLTQPPSASGTPGQRVTISCSGSSSNIGSNTVNWYQQLPGTAPKLLIYSN NQRPSGVPDRFSGSKSGTSASLAISGLQSEDEADYYCAAWDDSLNGYVFGT GTKLTVL (SEQ ID NO. 99) |
| YU1425-G11 | DVVMTQSPLSLPVTPGEPASISCRSSQSLLHSNGYNYLDWYLQKPGQSPQL LIYLGSNRASGVPDRFSGSGSGTDFTLKISRVEAEDVGVYYCMQALQTPLFG PGTKVDIK (SEQ ID NO. 93) |
| YU1425-C04 | QSALTQPASVSGSPGQSITISCTGTSSDVGGYNYVSWYQQHPGKAPKLMIY DVSNRPSGVSNRFSGSKSGNTASLTISGLQAEDEADYYCSSYTSSSTLVVFG GGTKLTVL (SEQ ID NO. 94) |
| YU1425-A01 | DVVMTQSPLSLPVTPGEPASISCRSSQSLLHSNGYNYLDWYLQKPGQSPQL LIYLGSNRASGVPDRFSGSGSGTDFTLKISRVEAEDVGVYYCMQALQTPYTF GQGTKVDIK (SEQ ID NO. 95) |
| YU1425-G09 | QSVLTQPPSVSAAPGQKVTISCSGSSSNIGNNYVSWYQQLPGTAPKLLIYDN NKRPSGIPDRFSGSKSGTSATLGITGLQTGDEADYYCGTWDSSLSAVVFGG GTKLTVL (SEQ ID NO. 97) |
| YU1425-H11 | DIQLTQSPSSLSASVGDRVTITCQASQDISNYLNWYQQKPGKAPKLLIYDASN LETGVPSRFSGSGSGTDFTFTISSLQPEDIATYYCQQYDNLPLTFGGGTKLEI K (SEQ ID NO. 98) |
| YU1425-F05 | QSVLTQPPSASGTPGQRVTISCSGSSSNIGSNYVYWYQQLPGTAPKLLIYRN SQRPSGVPDRFSGSKSGTSASLAISGLRSEDGADYYCAAWDDSLSVPYVFG TGTKLTVL (SEQ ID NO. 101) |
| YU1425-A12 | DIQLTQSPSTLSASVGDRVTITCRASQSISSWLAWYQQKPGKAPKLLIYKASS LESGVPSRFSGSGSGTEFTLTISSLQPDDFATYYCQQYNSYLITFGQGTRLEI K (SEQ ID NO. 102) |
| YU1425-B08 | QSVLTQPPSVSGAPGQRVTISCTGSSSNIGAGYDVHWYQQLPGTAPKLLIYG NSNRPSGVPDRFSGSKSGTSASLAITGLQAEDEADYYCQSYDSSLSGFVVF GGGTKLTVL (SEQ ID NO. 103) |
| YU1425-A04 | QSALTQPASVSGSPGQSITISCTGTSSDVGGYNYVSWYQQHPGKAPKLMIY DVSNRPSGVSNRFSGSKSGNTASLTISGLQAEDEADYYCSSYTSSSTVVFG GGTKLTVL (SEQ ID NO. 104) |
| YU1425-B05 | QSVLTQPPSVSGAPGQRVTISCTGSSSNIGAGYDVHWYQQLPGTAPKLLIYG NSNRPSGVPDRFSGSKSGTSASLAITGLQAEDEADYYCQSYDSSLSGSWVF GGGTKLTVL (SEQ ID NO. 105) |
| YU1425-C08 | SYVLTQPPSVSVSPGQTARITCSGDALPKQYAYWYQQKPGQAPVLVIYKDS ERPSGIPERFSGSSSGTTVTLTISGVQAEDEADYYCQSADSSGTYYVFGTGT KVTVL (SEQ ID NO. 106) |

| **Table 5: VH DNA sequences of exemplified antibodies** | |
|---|---|
| **Antibody** | **DNA of VH** |
| YU1425-A10 | GAAGTGCAGCTTGTTCAGAGCGGGGGAGGCCTCGTCCAACCTGGTCGAT CTTTGAGGCTTTCATGTGCAGCTTCAGGTTTTACCTTTGACGACTACGCT ATGCACTGGGTTCGCCAAGCTCCAGGTAAGGGTCTTGAATGGGTTTCAG |
| | GGATAAGTTGGAACTCTGGCTCCATCGGCTATGCAGACTCAGTCAAAGG CAGATTCACCATCTCCCGAGATAACGCCAAAAATAGTCTCTACCTTCAGA TGAACTCTCTCAGGGCCGAGGACACTGCATTGTATTATTGTGCTAAAGGC CTTGTTGCGGCAACTACACCGCCGTATTTCGATTACTGGGGGCAGGGTA CACTTGTCACTGTCTCAAGC (SEQ ID NO. 109) |
| YU1425-A09 | CAGGTTCAGCTGGTTCAAAGCGGCGGCGGGCTCGTGCAGCCGGGGCGA TCATTGAGACTGTCCTGTGCAGCGTCAGGTTTTACGTTCGACGATTATGC CATGCACTGGGTCCGGCAAGCACCAGGAAAAGGGCTCGAGTGGGTGTC TGGTATCTCCTGGAACTCTGGTAGTATCGGATATGCGGACAGTGTGAAAG GGCGATTCACAATTTCAAGGGATAATGCTAAAAACAGCCTCTACTTGCAA ATGAACAGCTTGAGAGCAGAAGATACAGCCCTGTACTATTGCGCTAAGGA TATATCTGGCTCACAACTGGTTTTGCTGGACTACTGGGGGCAAGGCACC ACCGTAACAGTGAGCTCT (SEQ ID NO. 110) |
| YU1425-E10 | GAGGTTCAACTGCTTGAGAGTGGAGGTGGTCTGGTACAGCCGGGACGAT CCTTGCGATTGAGCTGTGCAGCCACAGGATTCACCTTTGACGATTATGCC ATGCACTGGGTGAGACAGGCCCCTGGAAAAGGTTTGGAGTGGGTTAGCG GGATCAGTTGGAATTCCGGCTCAATAGGTTATGCCGATTCTGTAAAGGGA CGCTTTACGATCAGTAGAGACAATGCCAAAAACAGTCTCTACCTGCAAAT GAACTCACTTAGGGCAGAGGATACAGCCCTGTATTACTGTGCGAAAGCA AACTACTACGATAGTAGTGCCCTCGATTATTGGGGTCAAGGAACTCTCGT CACTGTCAGCAGC (SEQ ID NO. 111) |
| YU1425-C09 | GAAGTGCAGCTTCTCGAAAGCGGAGGGGGCCTGGTCCAACCTGGTGGC TCTCTCCGCCTCTCATGTAGCGCCTCCGGCTTCACCTTCTCCTCTTATGC AATGCACTGGGTGCGCCAGGCACCCGGAAAAGGCCTCGAATACGTTAGC GCTATTAGCTCCAACGGAGGAAGCACATATTACGCTGACAGTGTCAAAG GACGCTTTACCATAAGCCGCGATAACTCCAAGAATACCCTTTATTTGCAA ATGAGCTCACTCCGGGCAGAGGACACCGCAGTGTACTACTGCGTTAAAG GAGAACAATGGTTGGCCTACAATTGGTTTGATCCCTGGGGTCAAGGTACT ACGGTGACTGTTTCCAGT (SEQ ID NO. 112) |
| YU1425-C01 | CAAGTACAACTCGTACAATCCGGAGGAGGCCTGGTACAGCCTGGCGGTT CATTGCGGCTCTCCTGTGCAGCGTCTGGCTTTACGTTCTCATCCTATTCT ATGAGTTGGGTCCGCCAAGCGCCTGGCAAAGGCTTGGAGTGGGTATCAG CAATCAGTGGCTCTGGTGGAACATATTATGCAGATTCCGTGAAAGGGAG GTTCACTATTTCACGGGATTCTTCAAAAAACACGTTGTACCTCCAAATGAA CTCTTTGAGGGCAGAAGATACTGCCGTTTATTACTGCGCGCGAAGCCTCA GGGCGTTTTCTTACTGGGGCCAGGGGACTTTGGTTACTGTCTCTTCA (SEQ ID NO. 113) |
| YU1425-E01 | CAAGTTCAACTCGTGCAGTCTGGAGCGGAAGTTAAAAAACCAGGGGAAT CACTCAAGATATCTTGTAAGGGTTCTGGTTACAGTTTTACCTCATACTGGA TAGGGTGGGTTCGGCAAATGCCAGGCAAGGGACTGGAGTGGATGGGCA TTATCTATCCTGGAGATAGTGACACTCGCTACAGCCCATCTTTTCAGGGA CAGGTGACGATTTCCGCCGATAAAAGCATCTCAACGGCGTACCTTCAGT GGTCTAGCTTGAAAGCCAGCGATACTGCCATGTACTATTGCGCAAGGCT GGGGGTCAGCGGTTCTTACCGAAGATCAGGAATGGACGTTTGGGGTCAA GGGACCCTTGTAACTGTTAGTAGT (SEQ ID NO. 114) |
| YU1425-G02 | CAGGTCCAACTTGTGCAAAGCGGTGCCGAAGTTAAGAAGCCGGGGTCAT CAGTGAAGGTCAGCTGTAAAGCGTCTGGTGGAACATTTTCTAGCTATGCG ATCTCATGGGTGAGACAGGCTCCAGGGCAGGGCTTGGAGTGGATGGGC CGAATCATCCCAATTTTTGGAATTGCGAACTACGCACAAAAGTTTCAAGG GCGGGTCACCATTACAGCAGATAAATCAACATCAACGGCCTACATGGAAC TCTCTTCCCTGAGGTCTGAGGACACTGCCGTTTATTATTGTGCTCGAAGA TTTCAACACTGGGGACAGGGGACCTTGGTTACAGTTTCATCA (SEQ ID NO. 115) |
| YU1425-C04 | CAGGTACAGTTGGTCCAGTCAGGCGGGGGTCTTGTGCAACCAGGACGCT CACTCCGCCTTTCCTGCGCGGCGTCTGGCTTTACGTTTGATGATTACGCA ATGCACTGGGTAAGGCAGGCCCCAGGTAAAGGGCTTGAATGGGTTTCAG GTATTTCCTGGAATAGCGGAAGTATTGGCTACGCAGACTCTGTGAAGGG GCGGTTCACTATCAGTCGGGACAACGCTAAGAACTCCCTGTACTTGCAG ATGAATTCCCTGAGGGCGGAGGATACAGCTCTGTATTATTGTGCTAAAGA CAACTCTGGCAGTGGACGATATTTTGATTACTGGGGCCAAGGAACTCTCG TCACGGTTAGTAGC (SEQ ID NO. 116) |

| **Table 6: VL DNA sequences of exemplified antibodies** | |
|---|---|
| **Antibody** | **DNA of VL** |
| YU1425-A10 | CAATCCGCACTTACGCAACCGGCGAGCGTGAGTGGTTCACCTGGACAGA GTATCACTATTTCCTGTACGGGGACCAGTTCAGATGTTGGCGGATACAAC TACGTGTCATGGTACCAACAACACCCCGGGAAAGCCCCGAAATTGATGA TCTATGATGTCAGTAATCGCCCTTCAGGTGTATCCAACAGATTTTCAGGTT CTAAAAGCGGTAATACAGCATCACTCACCATTAGCGGGCTCCAAGCCGA GGACGAGGCAGATTACTACTGCTCCTCATATACTTCAAGCAGCACTCTGG TGTTCGGCGGAGGGACTAAAGTGACTGTTTTG (SEQ ID NO. 117) |
| YU1425-A09 | CAGAGTGTGTTGACTCAACCTCCCAGCGTTAGTGGGGCTCCGGGACAAC GGGTTACGATCAGTTGTACTGGATCATCATCCAACATCGGCGCCGGTTAC |
| | GACGTGCATTGGTATCAGCAGCTCCCAGGGACGGCTCCCAAACTTCTGA TATACGGTAATAGTAATAGGCCTTCAGGTGTACCTGACCGCTTCTCTGGC TCCAAATCAGGGACCAGTGCCTCTCTTGCTATCGCAGGTTTGCAAGCTGA GGATGAAGCTGACTATTATTGTCAAAGTTATGATAGTTCCCTTTCAGTCGT CTTTGGTGGCGGGACTAAGGTTACGGTATTG (SEQ ID NO. 118) |
| YU1425-E10 | CAGGTACAGTTGGTCCAGTCAGGCGGGGGTCTTGTGCAACCAGGACGCT CACTCCGCCTTTCCTGCGCGGCGTCTGGCTTTACGTTTGATGATTACGCA ATGCACTGGGTAAGGCAGGCCCCAGGTAAAGGGCTTGAATGGGTTTCAG GTATTTCCTGGAATAGCGGAAGTATTGGCTACGCAGACTCTGTGAAGGG GCGGTTCACTATCAGTCGGGACAACGCTAAGAACTCCCTGTACTTGCAG ATGAATTCCCTGAGGGCGGAGGATACAGCTCTGTATTATTGTGCTAAAGA CAACTCTGGCAGTGGACGATATTTTGATTACTGGGGCCAAGGAACTCTCG TCACGGTTAGTAGC (SEQ ID NO. 119) |
| YU1425-C09 | CAAAGCGTCCTTACTCAGCCCCCATCTGTGTCTGGAGCACCTGGTCAAC GGGTTACAATATCATGTACTGGATCAAGTTCAAACATAGGCGCCGGATAC GATGTGCATTGGTATCAACAGTTGCCGGGCACGGCGCCAAAGTTGCTTA TTTATGGTAACTCTAACAGACCTAGCGGCGTTCCAGACCGCTTCTCAGGC AGCAAGAGCGGCACAAGTGCGAGCCTGGCGATAACGGGACTGCAAGCC GAGGACGAAGCTGACTACTACTGCCAATCATATGATTCATCACTCTCAGG CTGGGGGGTATTTGGGGGAGGTACGAAACTGACTGTGCTC (SEQ ID NO. 120) |
| YU1425-C01 | GACGTTGTCATGACGCAGAGTCCCCTCTCCCTGCCAGTTACACCCGGCG AACCTGCTAGCATTAGTTGCAGAAGTTCTCAATCTCTTCTGCACTCTAATG GATATAATTACCTCGACTGGTATTTGCAAAAGCCGGGTCAGAGCCCACAG CTTCTCATATATCTGGGATCTAACCGGGCTTCTGGAGTCCCTGACCGGTT TTCCGGTTCCGGTTCTGGTACGGACTTTACCCTTAACATATCCCGCGTAG AAGCCGAAGATGTGGGAGTCTACTACTGTATGCAATCACTGAGGACCCC CCTGACCTTCGGCGGAGGAACTAAATTGGAGATCAAA (SEQ ID NO. 121) |
| YU1425-E01 | GAGATCGTGTTGACACAATCCCCGGGGACGTTGTCTCTCAGCCCTGGAG AGCGGGCAACTCTCAGTTGCCGAGCTTCTCAGTCCGTTTCCAGTAGCTAT CTCGCCTGGTACCAACAGAAGCCGGGTCAAGCTCCCCGGCTTCTGATTT ATGGCGCATCATCCAGAGCTACCGGCATCCCGGACCGATTCTCTGGCTC TGGCAGTGGTACTGATTTTACACTGACGATTAGCCGGTTGGAACCAGAG GATTTTGCCGTGTACTACTGCCAACAATACGGAAGCAGCCCGCTTACATT CGGGGGAGGTACAAAATTGGAGATCAAA (SEQ ID NO. 122) |
| YU1425-G02 | TCTTACGTTTTGACCCAGCCTCCCAGCGCGTCAGGCACGCCTGGACAGC GGGTGACCATATCCTGTAGTGGCTCTAGTAGCAATATAGGAAGCAACAC |
| | GGTAAATTGGTATCAACAGTTGCCCGGAACTGCACCCAAGCTGTTGATAT ATAGCAATAATCAAAGGCCGTCTGGAGTACCTGACCGGTTCTCAGGTAG CAAGAGTGGGACATCTGCATCATTGGCGATAAGTGGCCTTCAGTCAGAG GATGAGGCTGACTATTATTGTGCGGCTTGGGACGACTCCTTGAATGGCTA TGTATTTGGCACGGGGACCAAGCTGACGGTGCTC (SEQ ID NO. 123) |
| YU1425-C04 | CAGAGTGCGTTGACACAGCCCGCATCTGTGTCTGGTTCCCCGGGGCAGT CTATCACAATTTCATGCACCGGGACCTCCTCTGATGTCGGGGGATATAAT TATGTAAGTTGGTATCAACAGCACCCTGGCAAGGCCCCAAAGCTCATGAT TTATGACGTATCAAATAGACCATCTGGAGTGAGTAACAGATTTAGTGGCA GCAAAAGTGGGAACACGGCTTCCCTTACAATCAGCGGGTTGCAAGCCGA GGACGAAGCCGATTACTATTGTAGCAGTTACACTAGCAGCAGCACCCTG GTGGTTTTTGGGGGGGGCACCAAATTGACGGTCCTT (SEQ ID NO. 124) |

| **Table 7: Constant region amino acid sequences** | |
|---|---|
| **AA of CH** |  (SEQ ID NO. 125) |
| **AA of CL** | GQPKAAPSVTLFPPSSEELQANKATLVCLISDFYPGAVTVAWKADSSPVKAG VETTTPSKQSNNKYAASSYLSLTPEQWKSHRSYSCQVTHEGSTVEKTVAPT ECS (SEQ ID NO. 126) |

| **Table 8: List of mAbs produced and tested in vitro** | | | | |
|---|---|---|---|---|
| **Antibody** | **Batch** | **Conc (mg/ml)** | **Total protein (mg)** | **Yield (mg/L)** |
| YU1425-A10 | Ypr-2024-8-21 | 0,62 | 1,80 | 35,96 |
| YU1425-A09 | Ypr-2024-11-46 | 0,34 | 1,96 | 39,25 |
| YU1425-E10 | Ypr-2024-10-7 | 0,28 | 1,13 | 22,67 |
| YU1425-C09 | Ypr-2024-11-47 | 0,59 | 3,42 | 68,44 |
| YU1425-C01 | Ypr-2024-8-18 | 0,60 | 1,74 | 34,87 |
| YU1425-E01 | Ypr-2024-8-19 | 0,95 | 2,76 | 55,16 |
| YU1425-G02 | Ypr-2024-10-8 | 0,20 | 0,78 | 15,7 |
| YU1425-C04¹ | Ypr-2024-10-5 | 0,04 | 0,17 | 3,4 |

| | | | | |
|---|---|---|---|---|
| ¹ mAb YU1425-C04 has not been further tested in vitro. | | | | |

| **Table 9: Binding affinity of anti-IGFBP7 mAbs to IGFBP7 (murine, human and Strept human)** | | | | |
|---|---|---|---|---|
| **Antibody** | **Antigen** | **EC50 (ng/ml)** | **95% CI EC50 (ng/ml)** | **R squared** |
| YU1425-A10 (Ypr-2024-8-21) | Strep+human IGFBP7-bio² | 74.03 | 66.99 to 81.69 | 0.9987 |
| | Human IGFBP7 | 39.54 | 29.14 to 52.31 | 0.9845 |
| | Mouse IGFBP7 | 416.7 | 339.2 to 512.0 | 0.9954 |
| YU 1425-A09 (Ypr-2024-11-46) | Strep+human IGFBP7-bio | 1248 | 800.6 to 1910 | 0.9763 |
| | Human IGFBP7 | 455.2 | 417.8 to 495.8 | 0.9991 |
| | Mouse IGFBP7 | 813 | 733.2 to 901.4 | 0.9986 |
| YU1425-E10 (Ypr-2024-10-7) | Strep+human IGFBP7-bio | 532.2 | 446.3 to 633.2 | 0.996 |
| | Human IGFBP7 | 257.9 | 214.1 to 311.0 | 0.996 |
| | Mouse IGFBP7 | 373.8 | 335.1 to 416.8 | 0.9985 |
| YU1425-C09 (Ypr-2024-11-47) | Strep+human IGFBP7-bio | 2929 | 2069 to 4084 | 0.9786 |
| | Human IGFBP7 | 360.7 | 327.5 to 397.1 | 0.9987 |
| | Mouse IGFBP7 | 201.9 | 175.9 to 231.5 | 0.9977 |
| YU1425-C01 (Ypr-2024-8-18) | Strep+human IGFBP7-bio | 3071 | 2382 to 3941 | 0.9884 |
| | Human IGFBP7 | 3614 | 2864 to 4538 | 0.9892 |
| | Mouse IGFBP7 | 5230 | 2562 to 9505 | 0.9168 |
| YU1425-E01 (Ypr-2024-8-19) | Strep+human IGFBP7-bio | 2283 | 1650 to 3139 | 0.9844 |
| | Human IGFBP7 | 198.3 | 181.3 to 216.7 | 0.9989 |
| | Mouse IGFBP7 | 445.5 | 386.8 to 513.3 | 0.9976 |
| YU1425-G02 (Ypr-2024-10-8) | Strep+human IGFBP7-bio | 197.4 | 139.2 to 278.2 | 0.9883 |
| | Human IGFBP7 | 74.61 | 46.43 to 115.5 | 0.9769 |
| | Mouse IGFBP7 | 365.1 | 277.3 to 480.5 | 0.9921 |
| YU1425-C04¹ (Ypr-2024-10-5) | Strep+human IGFBP7-bio | 2299 | 1594 to 3260 | 0.9794 |
| | Human IGFBP7 | 1225 | 990.7 to 1511 | 0.994 |
| | Mouse IGFBP7 | 1311 | 615.8 to 2625 | 0.9386 |

| | | | | |
|---|---|---|---|---|
| mAbs with best EC50 are highlighted in grey. ¹ mAb YU1425-C04 has not been further tested in vitro. ² bio stands for biotinylated. | | | | |

### BIBLIOGRAPHY

Akaogi, K., Okabe, Y., Sato, J., Nagashima, Y., Yasumitsu, H., Sugahara, K., & Miyazaki, K. (1996). Specific accumulation of tumor-derived adhesion factor in tumor blood vessels and in capillary tube-like structures of cultured vascular endothelial cells. Proceedings of the National Academy of Sciences of the United States of America, 93(16), 8384-8389. https://doi.org/10.1073/PNAS.93.16.8384
Bai, Z., Fang, F., Xu, Z., Lu, C., Wang, X., Chen, J., Pan, J., Wang, J., Li, Y. Serum and urine FGF23 and IGFBP-7 for the prediction of acute kidney injury in critically ill children. BMC Pediatr. 2018 Jun 15;18(1):192. doi: 10.1186/s12887-018-1175-y
Bracun, V., van Essen, B., Voors, A. A., van Veldhuisen, D. J., Dickstein, K., Zannad, F., Metra, M., Anker, S., Samani, N. J., Ponikowski, P., Filippatos, G., Cleland, J. G. F., Lang, C. C., Ng, L. L., Shi, C., de Wit, S., Aboumsallem, J. P., Meijers, W. C., Klip, Ij. T., ... de Boer, R. A. (2022). Insulin-like growth factor binding protein 7 (IGFBP7), a link between heart failure and senescence. ESC Heart Failure, 9(6), 4167. https://doi.org/10.1002/EHF2.14120
Chugh, S., Ouzounian, M., Lu, Z., Mohamed, S., Li, W., Bousette, N., Liu, P. P., & Gramolini, A. O. (2013). Pilot study identifying myosin heavy chain 7, desmin, insulin-like growth factor 7, and annexin A2 as circulating biomarkers of human heart failure. Proteomics, 13(15), 2324-2334. https://doi. org/10.1002/PM I C.201200455
Evdokimova V, Tognon CE, Benatar T, Yang W, Krutikov K, Pollak M, Sorensen PH, Seth A. IGFBP7 binds to the IGF-1 receptor and blocks its activation by insulin-like growth factors. Sci Signal. 2012 Dec 18;5(255):ra92. doi: 10.1126/scisignal.2003184
Jourde-Chiche, N., Fakhouri, F., Dou, L., Bellien, J., Burtey, S., Frimat, M., Jarrot, P. A., Kaplanski, G., Le Quintrec, M., Pernin, V., Rigothier, C., Sallée, M., Fremeaux-Bacchi, V., Guerrot, D., & Roumenina, L. T. (2019). Endothelium structure and function in kidney health and disease. Nature Reviews. Nephrology, 15(2), 87-108. https://doi.org/10.1038/S41581-018-0098-Z
Kashani K, Al-Khafaji A, Ardiles T, Artigas A, Bagshaw SM, Bell M, Bihorac A, Birkhahn R, Cely CM, Chawla LS, Davison DL, Feldkamp T, Forni LG, Gong MN, Gunnerson KJ, Haase M, Hackett J, Honore PM, Hoste EA, Joannes-Boyau O, Joannidis M, Kim P, Koyner JL, Laskowitz DT, Lissauer ME, Marx G, McCullough PA, Mullaney S, Ostermann M, Rimmelé T, Shapiro NI, Shaw AD, Shi J, Sprague AM, Vincent JL, Vinsonneau C, Wagner L, Walker MG, Wilkerson RG, Zacharowski K, Kellum JA. Discovery and validation of cell cycle arrest biomarkers in human acute kidney injury. Crit Care. 2013 Feb 6;17(1):R25. doi: 10.1186/cc12503.
Koyner JL, Shaw AD, Chawla LS, Hoste EA, Bihorac A, Kashani K, Haase M, Shi J, Kellum JA; Sapphire Investigators. Tissue Inhibitor Metalloproteinase-2 (TIMP-2)·IGF-Binding Protein-7 (IGFBP7) Levels Are Associated with Adverse Long-Term Outcomes in Patients with AKI. J Am Soc Nephrol. 2015 Jul;26(7):1747-54. doi: 10.1681/ASN.2014060556.
Li, Q., Shao, S., Zhu, Z., Chen, J., Hao, J., Bai, Y., Li, B., Dang, E., & Wang, G. (2023). An IGFBP7hi endothelial cell subset drives T cell extravasation in psoriasis via endothelial glycocalyx degradation. The Journal of Clinical Investigation, 133(9). https://doi.ora/10.1172/JCI160451
Li Y, Fu L, Wu B, Guo X, Shi Y, Lv C, Yu Y, Zhang Y, Liang Z, Zhong C, Han S, Xu F, Tian Y. Angiogenesis modulated by CD93 and its natural ligands IGFBP7 and MMRN2: a new target to facilitate solid tumor therapy by vasculature normalization. Cancer Cell Int. 2023 Sep 2;23(1):189. doi: 10.1186/s12935-023-03044-z
Motiwala, S. R., Szymonifka, J., Belcher, A., Weiner, R. B., Baggish, A. L., Gaggin, H. K., Bhardwaj, A., & Januzzi, J. L. (2014). Measurement of novel biomarkers to predict chronic heart failure outcomes and left ventricular remodeling. Journal of Cardiovascular Translational Research, 7(2), 250-261. https://doi.org/10.1007/S12265-013-9522-8
Oh, Y., Nagalla, S. R., Yamanaka, Y., Kim, H. S., Wilson, E., & Rosenfeld, R. G. (1996). Synthesis and characterization of insulin-like growth factor-binding protein (IGFBP)-7. Recombinant human mac25 protein specifically binds IGF-I and -II. The Journal of Biological Chemistry, 271(48), 30322-30325. https://doi.org/10.1074/JBC.271.48.30322
Shibata, Y., Tsukazaki, T., Hirata, K., Xin, C., & Yamaguchi, A. (2004). Role of a new member of IGFBP superfamily, IGFBP-rP10, in proliferation and differentiation of osteoblastic cells. Biochemical and Biophysical Research Communications, 325(4), 1194-1200. https://doi.org/10.1016/J.BBRC.2004.10.157
St. Croix, B., Rago, C., Velculescu, V., Traverso, G., Romans, K. E., Montegomery, E., Lal, A., Riggins, G. J., Lengauer, C., Vogelstein, B., & Kinzler, K. W. (2000). Genes expressed in human tumor endothelium. Science (New York, N.Y.), 289(5482), 1197-1202. https://doi.ora/10.1126/SCIENCE.289.5482.1197
Swisshelm, K., Ryan, K., Tsuchiya, K., & Sager, R. (1995). Enhanced expression of an insulin growth factor-like binding protein (mac25) in senescent human mammary epithelial cells and induced expression with retinoic acid. Proceedings of the National Academy of Sciences of the United States of America, 92(10), 4472-4476. https://doi.org/10.1073/PNAS.92.10.4472
van Eikema Hommes, N. J. R., Saalfrank, R. W., Puchta, R., & Seitz, V. (2000). A secreted tumor-suppressor, mac25, with activin-binding activity. Molecular Medicine (Cambridge, Mass.), 6(2), 126-132. https://doi.org/10.1007/S0089400060126
Xie, Y., Ankawi, G., Yang, B., Garzotto, F., Passannante, A., Breglia, A., Digvijay, K., Ferrari, F., Brendolan, A., Raffaele, B., Giavarina, D., Gregori, D., & Ronco, C. (2019). Tissue inhibitor metalloproteinase-2 (TIMP-2) • IGF-binding protein-7 (IGFBP7) levels are associated with adverse outcomes in patients in the intensive care unit with acute kidney injury. Kidney International, 95(6), 1486-1493. https://doi.org/10.1016/J.KINT.2019.01.020
Yamanaka, Y., Wilson, E. M., Rosenfeld, R. G., & Oh, Y. (1997). Inhibition of insulin receptor activation by insulin-like growth factor binding proteins. The Journal of Biological Chemistry, 272(49), 30729-30734. https://doi.org/10.1074/JBC.272.49.30729
Yu, J. tao, Hu, X. wei, Yang, Q., Shan, R. run, Zhang, Y., Dong, Z. hui, Li, H. di, Wang, J. nan, Li, C., Xie, S. shuai, Dong, Y. hang, Ni, W. jian, Jiang, L., Liu, X. qi, Wei, B., Wen, J. gen, Liu, M. ming, Chen, Q., Yang, Y. ru, ... Meng, X. ming. (2022). Insulin-like growth factor binding protein 7 promotes acute kidney injury by alleviating poly ADP ribose polymerase 1 degradation. Kidney International, 102(4), 828-844. https://doi.org/10.1016/J.KINT.2022.05.026
Zhang, L., Smyth, D., Al-Khalaf, M., Blet, A., Du, Q., Bernick, J., Gong, M., Chi, X., Oh, Y., Roba-Oshin, M., Coletta, E., Feletou, M., Gramolini, A. O., Kim, K. H., Coutinho, T., Januzzi, J. L., Tyl, B., Ziegler, A., & Liu, P. P. (2022). Insulin-like growth factor-binding protein-7 (IGFBP7) links senescence to heart failure. Nature Cardiovascular Research 2022 1:12, 1(12), 1195-1214. https://doi.org/10.1038/s44161-022-00181-y

## Claims

1. An isolated antibody or antigen binding fragment or variant thereof that binds to IGFBP7 and/or receptor thereof, said isolated antibody or antigen binding fragment thereof comprising:
a. a heavy chain variable domain (VH) comprising:
i. a CDR1 sequence of the amino acid sequence selected from the group consisting of: SEQ ID NO: 1, 14, 29, 32, 9, 20, 24 and 26;
ii. a CDR2 sequence of the amino acid sequence selected from the group consisting of: SEQ ID NO: 2, 15, 30, 33, 13, 10, 17, 21 and 27; and
iii. a CDR3 sequence of the amino acid sequence selected from the group consisting of: SEQ ID NO: 8, 4, 3, 16, 31, 34, 128, 5, 6, 7, 11, 12, 18, 19, 22, 23, 25 and 28; and/or
b. a light chain variable domain (VL) comprising:
i. a CDR1 sequence of the amino acid sequence selected from the group consisting of: SEQ ID NO: 35, 38, 41, 70, 53, 47, 50, 57, 60 and 66;
ii. a CDR2 sequence of the amino acid sequence selected from the group consisting of: SEQ ID NO: 36, 39, 42, 71, 54, 48, 51, 58, 61 and 67; and
iii. a CDR3 sequence of the amino acid sequence selected from the group consisting of: SEQ ID NO: 46, 40, 37, 56, 69, 72, 55, 43, 44, 45, 49, 52, 59, 62, 63, 64, 65 and 68; and
wherein CDRs are according to Kabat.

2. An isolated antibody or antigen binding fragment or variant thereof that binds to IGFBP7 and/or receptor thereof according to claim 1, said isolated antibody or antigen binding fragment thereof comprising:
a. a heavy chain variable domain (VH) comprising:
i. a CDR1 sequence of the amino acid sequence selected from the group consisting of: SEQ ID NO: 1, 14, 29, and 32;
ii. a CDR2 sequence of the amino acid sequence selected from the group consisting of: SEQ ID NO: 2, 15, 30 and 33; and
iii. a CDR3 sequence of the amino acid sequence selected from the group consisting of: SEQ ID NO: 8, 4, 3, 16, 31 and 34; and/or
b. a light chain variable domain (VL) comprising:
i. a CDR1 sequence of the amino acid sequence selected from the group consisting of: SEQ ID NO: 35, 38, 41 and 70;
ii. a CDR2 sequence of the amino acid sequence selected from the group consisting of: SEQ ID NO: 36, 39, 42 and 71; and
iii. a CDR3 sequence of the amino acid sequence selected from the group consisting of: SEQ ID NO: 46, 40, 37, 56, 69 and 72; and
wherein CDRs are according to Kabat.

3. The isolated antibody or antigen binding fragment thereof according to claims 1 and 2 comprising as CDRs:
SEQ ID NO: 1 and SEQ ID NO: 2 and SEQ ID NO: 8 and SEQ ID NO: 35 and SEQ ID NO: 36 and SEQ ID NO: 46 of YU1425-A10; or
SEQ ID NO: 1 and SEQ ID NO: 2 and SEQ ID NO: 4 and SEQ ID NO: 38 and SEQ ID NO: 39 and SEQ ID NO: 40 of YU1425-A09; or
SEQ ID NO: 1 and SEQ ID NO: 2 and SEQ ID NO: 3 and SEQ ID NO: 35 and SEQ ID NO: 36 and SEQ ID NO: 37 of YU1425-E10; or
SEQ ID NO: 14 and SEQ ID NO: 15 and SEQ ID NO: 16 and SEQ ID NO: 38 and SEQ ID NO: 39 and SEQ ID NO: 56 of YU1425-C09; or
SEQ ID NO: 29 and SEQ ID NO: 30 and SEQ ID NO: 31 and SEQ ID NO: 41 and SEQ ID NO: 42 and SEQ ID NO: 69 of YU1425-C01; or
SEQ ID NO: 32 and SEQ ID NO: 33 and SEQ ID NO: 34 and SEQ ID NO: 70 and SEQ ID NO: 71 and SEQ ID NO: 72 of YU1425-E01.

4. The isolated antibody or antigen binding fragment thereof according to claim 3 comprising:
a. a heavy chain variable domain (VH) comprising:
- a CDR1 comprising or consisting of the amino acid sequence of SEQ ID NO: 1;
- a CDR2 comprising or consisting of the amino acid sequence SEQ ID NO: 2;
- a CDR3 comprising or consisting of the amino acid sequence of SEQ ID NO: 8; and
b. a light chain variable domain (VL) comprising:
- CDR1 comprising or consisting of the amino acid sequence of SEQ ID NO: 35;
- CDR2 comprising or consisting of the amino acid sequence of SEQ ID NO: 36; and
- CDR3 comprising or consisting of the amino acid sequence of SEQ ID NO: 46;
or comprising:
a. a heavy chain variable domain (VH) comprising:
- CDR1 comprising or consisting of the amino acid sequence of SEQ ID NO: 1;
- CDR2 comprising or consisting of the amino acid sequence of SEQ ID NO: 2;
- CDR3 comprising or consisting of the amino acid sequence of SEQ ID NO: 4; and
b. a light chain variable domain (VL) comprising:
- CDR1 comprising or consisting of the amino acid sequence of SEQ ID NO: 38;
- CDR2 comprising or consisting of the amino acid sequence of SEQ ID NO: 39; and
- CDR3 comprising or consisting of the amino acid sequence of SEQ ID NO: 40;
or comprising:
a. a heavy chain variable domain (VH) comprising:
- CDR1 comprising or consisting of the amino acid sequence of SEQ ID NO: 1;
- CDR2 comprising or consisting of the amino acid sequence of SEQ ID NO: 2;
- CDR3 comprising or consisting of the amino acid sequence of SEQ ID NO: 3; and
b. a light chain variable domain (VL) comprising:
- CDR1 comprising or consisting of the amino acid sequence of SEQ ID NO: 35;
- CDR2 comprising or consisting of the amino acid sequence of SEQ ID NO: 36;
- CDR3 comprising or consisting of the amino acid sequence of SEQ ID NO: 37;
or comprising:
a. a heavy chain variable domain (VH) comprising:
- CDR1 comprising or consisting of the amino acid sequence of SEQ ID NO: 14;
- CDR2 comprising or consisting of the amino acid sequence of SEQ ID NO: 15;
- CDR3 comprising or consisting of the amino acid sequence of SEQ ID NO: 16; and
b. a light chain variable domain (VL) comprising:
- CDR1 comprising or consisting of the amino acid sequence of SEQ ID NO: 38;
- CDR2 comprising or consisting of the amino acid sequence of SEQ ID NO: 39; and
- CDR3 comprising or consisting of the amino acid sequence of SEQ ID NO: 56;
or comprising:
a. a heavy chain variable domain (VH) comprising:
- CDR1 comprising or consisting of the amino acid sequence of SEQ ID NO: 29;
- CDR2 comprising or consisting of the amino acid sequence of SEQ ID NO: 30;
- CDR3 comprising or consisting of the amino acid sequence of SEQ ID NO: 31; and
b. a light chain variable domain (VL) comprising:
- CDR1 comprising or consisting of the amino acid sequence of SEQ ID NO: 41;
- CDR2 comprising or consisting of the amino acid sequence of SEQ ID NO: 42; and
- CDR3 comprising or consisting of the amino acid sequence of SEQ ID NO: 69;
or comprising:
a. a heavy chain variable domain (VH) comprising:
- CDR1 comprising or consisting of the amino acid sequence of SEQ ID NO: 32;
- CDR2 comprising or consisting of the amino acid sequence of SEQ ID NO: 33;
- CDR3 comprising or consisting of the amino acid sequence of SEQ ID NO: 34; and
b. a light chain variable domain (VL) comprising:
- CDR1 comprising or consisting of the amino acid sequence of SEQ ID NO: 70;
- CDR2 comprising or consisting of the amino acid sequence of SEQ ID NO: 71; and
- CDR3 comprising or consisting of the amino acid sequence of SEQ ID NO: 72;
wherein each CDR is defined according to Kabat.

5. The isolated antibody or antigen binding fragment thereof according to any one of previous claims comprising:
a. a heavy chain variable domain sequence having at least 80 %, or 85 %, or 90%, or 95%, or 100% identity to the amino acid sequence selected from the group consisting of: SEQ ID NO: 78, 74, 73, 82, 89 and 90;
b. a light chain variable domain sequence having at least 80 %, or 85 %, or 90%, or 95%, or 100% identity to the amino acid sequence selected from the group consisting of: SEQ ID NO: 96, 92, 91, 100, 107 and 108; or
c. the heavy chain variable domain of (a) and the light chain variable domain of (b).

6. The isolated antibody or antigen binding fragment thereof according to claim 5 comprising:
a heavy chain variable domain comprising or consisting of a sequence having at least 80 %, or 85 %, or 90%, or 95%, or 100% identity to SEQ ID NO: 78 and a light chain variable domain comprising or consisting of a sequence having at least 80 %, or 85 %, or 90%, or 95%, or 100% identity to SEQ ID NO: 96; or
a heavy chain variable domain comprising or consisting of a sequence having at least 80 %, or 85 %, or 90%, or 95%, or 100% identity to SEQ ID NO: 74 and a light chain variable domain comprising or consisting of a sequence having at least 80%, or 85 %, or 90%, or 95%, or 100% identity to SEQ ID NO: 92; or
a heavy chain variable domain comprising or consisting of a sequence having at least 80%, or 85 %, or 90%, or 95%, or 100% identity to SEQ ID NO: 73 and a light chain variable domain comprising or consisting of a sequence having at least 80%, or 85 %, or 90%, or 95%, or 100% identity to SEQ ID NO: 91; or
a heavy chain variable domain comprising or consisting of a sequence having at least 80%, or 85 %, or 90%, or 95%, or 100% identity to SEQ ID NO: 82 and a light chain variable domain comprising or consisting of a sequence having at least 80%, or 85 %, or 90%, or 95%, or 100% identity to SEQ ID NO: 100; or
a heavy chain variable domain comprising or consisting of a sequence having at least 80%, or 85 %, or 90%, or 95%, or 100% identity to SEQ ID NO: 89 and a light chain variable domain comprising or consisting of a sequence having at least 80%, or 85 %, or 90%, or 95%, or 100% identity to SEQ ID NO: 107; or
a heavy chain variable domain comprising or consisting of a sequence having at least 80%, or 85 %, or 90%, or 95%, or 100% identity to SEQ ID NO: 90 and a light chain variable domain comprising or consisting of a sequence having at least 80%, or 85 %, or 90%, or 95%, or 100% identity to SEQ ID NO: 108.

7. The isolated antibody or antigen binding fragment thereof according to any one of previous claims comprising a heavy chain variable domain encoded by a nucleotide sequence selected from the group consisting of: SEQ ID NO: 109, SEQ ID NO: 110, SEQ ID NO: 111, SEQ ID NO: 112, SEQ ID NO: 113 and SEQ ID NO: 114; and/or a light chain variable domain encoded by a nucleotide sequence selected from the group consisting of: SEQ ID NO: 117, SEQ ID NO: 118, SEQ ID NO: 119, SEQ ID NO: 120, SEQ ID NO: 121 and SEQ ID NO: 122.

8. The isolated antibody according to any one of previous claims selected from the group consisting of: YU1425-A10, YU1425-A09, YU1425-E10, YU1425-C09, YU1425-C01 and YU1025-E01, preferably it is the antibody YU1425-A10 or YU1425-A09 or YU1425-E10, or an isolated antibody or antigen binding fragment thereof that:
(a) binds specifically to an epitope on IGFBP7, said epitope being the same or similar epitope as the epitope recognized by the monoclonal antibody YU1425-E10, YU1425-A09, YU1425-A10, YU1425-C09, YU1425-C01 or YU1025-E01; or
(b) cross-competes for binding with the monoclonal antibody YU1425-E10, YU1425-A09, YU1425-A10, YU1425-C09, YU1425-C01 or YU1025-E01; or
(c) shows the same or similar binding affinity or specificity, or both, as any of monoclonal antibodies YU1425-E10, YU1425-A09, YU1425-A10, YU1425-C09, YU1425-C01 or YU1025-E01; or
(d) has one or more biological properties of an antibody molecule chosen from YU1425-E10, YU1425-A09, YU1425-A10, YU1425-C09, YU1425-C01 or YU1025-E01; and/or
(e) has one or more pharmacokinetic properties of an antibody molecule chosen from YU1425-E10, YU1425-A09, YU1425-A10, YU1425-C09, YU1425-C01 or YU1025-E01.

9. The isolated antibody according to any one of previous claims being a human or a humanized antibody and/or being an IgG1 antibody, preferably an IgG1 kappa antibody, an IgG1 lambda antibody.

10. The isolated antibody according to any one of previous claims comprising a heavy chain constant region comprising or consisting of a sequence with SEQ ID NO. 125 or having at least 80%, or 85 %, or 90%, or 95%, or 100% identity to SEQ ID No. 125 and/or a light chain constant region comprising or consisting of a sequence with SEQ ID NO. 126, or having at least 80%, or 85 %, or 90%, or 95%, or 100% identity to SEQ ID No. 126 preferably wherein said heavy chain constant region is a human IgG1.

11. An isolated polynucleotide comprising at least one sequence that encodes the antibody or antigen binding fragment thereof according to any one of previous claims, preferably said polynucleotide being a cDNA, preferably said polynucleotide comprising or consisting of one or more nucleotide sequences selected from the group consisting of SEQ ID NO: 109 to SEQ ID NO: 114 and SEQ ID NO: 117 to SEQ ID NO: 122.

12. A vector comprising the isolated polynucleotide of claim 11, preferably said vector being selected from the group consisting of a plasmid, a viral vector, a non-episomal mammalian vector, an expression vector and a recombinant expression vector, or an isolated cell comprising said isolated polynucleotide of claim 11 or said vector of claim 12, preferably said isolated cell being a hybridoma or a Human Embryonic Kidney cell (HEK293).

13. A pharmaceutical composition comprising the antibody or antigen binding fragment thereof according to any one of claims 1 to 10 or the polynucleotide of claim 11 or the vector or the isolated cell of claim 12 and at least one pharmaceutically acceptable carrier, preferably said composition further comprising a second therapeutic agent.

14. The antibody or antigen binding fragment thereof according to any one of claims 1 to 10 or the polynucleotide of claim 11 or the vector or the isolated cell of claim 12 or the pharmaceutical composition of claim 13 for use as a medicament.

15. The antibody or antigen binding fragment thereof according to any one of claims 1 to 10 or the polynucleotide of claim 11 or the vector or the isolated cell of claim 12 or the pharmaceutical composition of claim 13 for use in the treatment and/or prevention of vascular stress-mediated senescence, inflammation, and fibrosis or fibrotic condition, and acute stress cellular damage, preferably wherein said vascular stress-mediated senescence, inflammation, and fibrosis or fibrotic condition is selected from psoriasis, heart failure or vasculopathy; and wherein said acute stress cellular damage is acute kidney injury.
